(19) Europäisches Patentamt
European Patent Office
Office européen des brevets

(11) EP 4 755 876 A1

# (12) EUROPEAN PATENT APPLICATION

(43) Date of publication:
**10.06.2026 Bulletin 2026/24**

(21) Application number: **24217166.8**

(22) Date of filing: **03.12.2024**

(51) International Patent Classification (IPC):
***C07D 251/24*** *(2006.01)* ***H10K 50/10*** *(2023.01)*

(52) Cooperative Patent Classification (CPC):
**C07D 251/24; H10K 50/10; H10K 50/11**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Novaled GmbH**
**01099 Dresden (DE)**

(72) Inventors:
- **STENNETT, Thomas**
  **01099 Dresden (DE)**
- **SCHOLZ, Johannes**
  **01099 Dresden (DE)**
- **GANIER, Jerome**
  **01099 Dresden (DE)**
- **SCHULZE, Benjamin**
  **01099 Dresden (DE)**

(74) Representative: **Erbacher, Martin**
**Boehmert & Boehmert**
**Anwaltspartnerschaft mbB**
**Pettenkoferstrasse 22**
**80336 München (DE)**

## (54) COMPOUND WITH A TRIAZINE-PHENYL-TRIAZINE/PYRIMIDINE CORE, ORGANIC LIGHT EMITTING DIODE, AND DISPLAY DEVICE

(57) The present invention relates to a compound. The present invention further relates to an organic light emitting diode comprising the compound, to a display device comprising the organic light emitting diode, and to a process for preparing the organic light emitting diode.

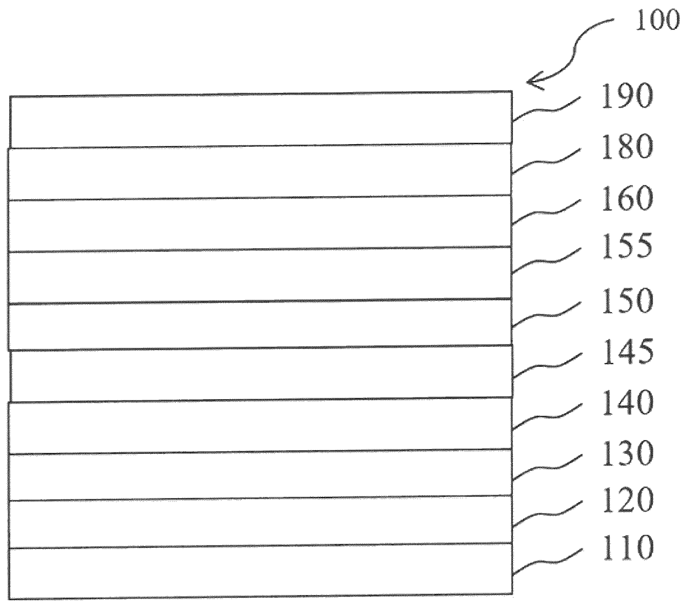


Fig. 1

EP 4 755 876 A1

## Description

TECHNICAL FIELD

The present invention relates to a compound. The present invention further relates to an organic light emitting diode comprising the compound, to a display device comprising the organic light emitting diode, and to a process for preparing the organic light emitting diode.

BACKGROUND OF THE INVENTION

Organic semiconducting devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing thermal stability is needed so that the organic semiconducting device, such as an organic light emitting diode, may be applied to a large-size flat panel display.

It is, therefore, the object of the present invention to provide organic light emitting diodes overcoming drawbacks of the prior art, in particular providing organic light emitting diodes with better material properties such as rate onset temperature (TRO) and glass transition temperature (Tg), better performance of the organic light emitting diode, in particular improved efficiency at comparable voltage.

DISCLOSURE

This object is achieved by a compound of formula (I)

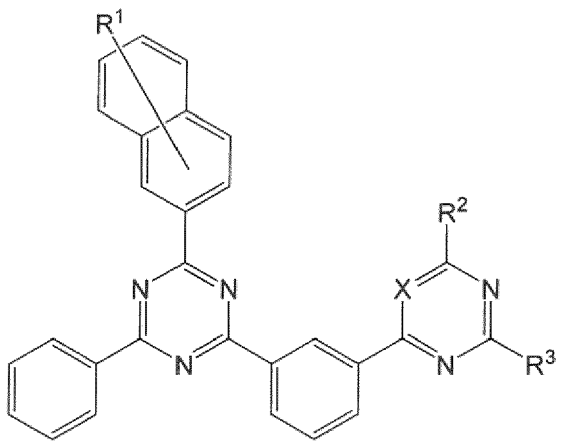

(I)

wherein

- Xis CH or N;
- $R^1$ is selected from the group consisting of H, phenyl, biphenyl, pyridyl, naphthyl, dibenzofuranyl, and dibenzothiophenyl;
- $R^2$ and $R^3$ are independently $C_6$ to $C_{12}$;

- at least one of $R^2$ and $R^3$ is phenyl; and
- the compound of formula (I) may be unsubstituted or substituted with one or more substituents independently selected from the group consisting of D, $C_1$ to $C_6$ alkyl; $C_1$ to $C_6$ alkoxy; and $SiR'_3$, wherein R' is independently selected from $C_1$ to $C_6$ alkyl.

This object is further achieved by an organic light emitting diode comprising an anode, a cathode, and an organic semiconducting layer, wherein

- the organic semiconducting layer is arranged between the anode and the cathode; and
- the organic semiconducting layer comprises the compound of formula (I) according to the present invention.

This object is further achieved by a display device comprising the organic light emitting diode according to the present invention.

Surprisingly, it was found that the organic light emitting diodes and display devices according to the present invention have improved properties in comparison with respective devices of the prior art, especially having improved efficiency at comparable operating voltage.

Compound of formula (I)

The present invention relates to a compound of formula (I)

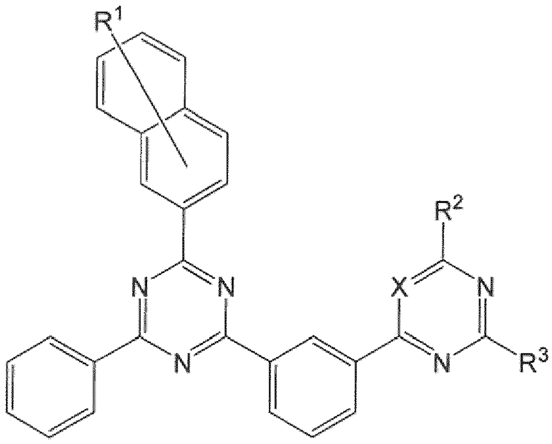

(I).

In accordance with the present disclosure, as an illustrative example using an arbitrary group A in a formula showing the following binding situation,

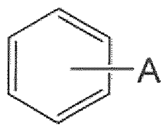

the group A may be bound to any suitable binding position.

That is, in formula (I), bonding of $R^1$ to

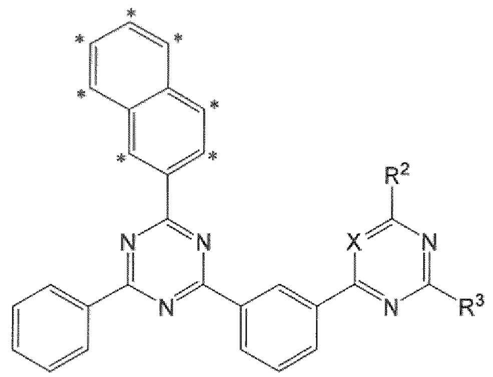

may be at any position labelled with "*", for example at

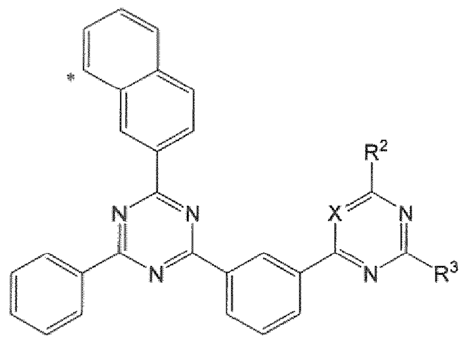

to form the structure

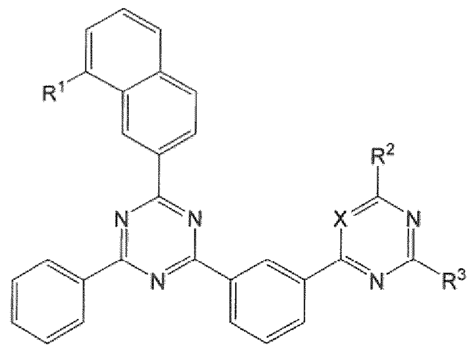

$R^1$ may be bound to the following positions labeled with "*"

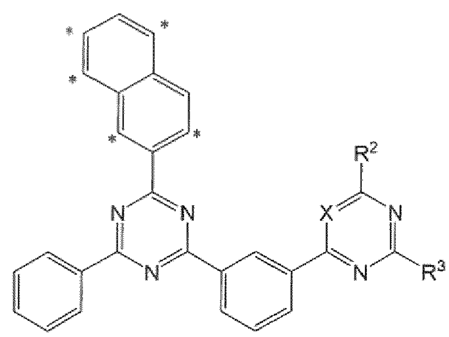

$R^1$ may be bound to the following positions labeled with "*"

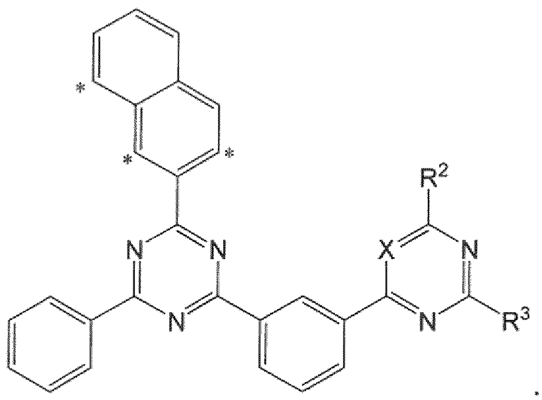

$R^1$ may be bound to the following positions labeled with "*"

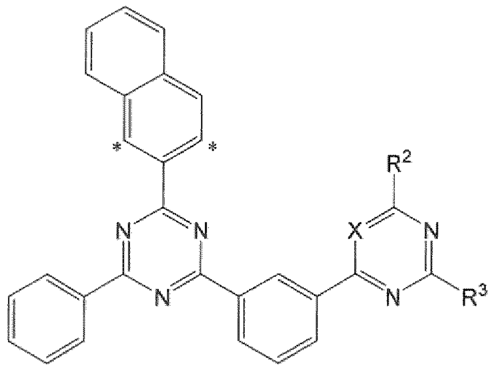

$R^1$ may be bound to the following position labeled with "*"

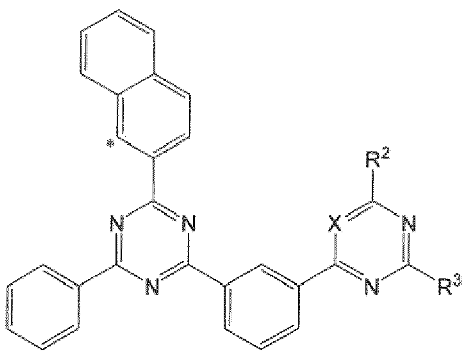

In formula (I), X is CH or N. X may be N.

In formula (I), $R^1$ is selected from the group consisting of H, phenyl, biphenyl, pyridyl, naphthyl, dibenzofuranyl, and dibenzothiophenyl.

$R^1$ may be selected from the group consisting of phenyl, biphenyl, pyridyl, alkyl, naphthyl, dibenzofuranyl, and dibenzothiophenyl.

$R^1$ may be selected from the group consisting of H, phenyl, biphenyl, pyridyl, and dibenzofuranyl.

$R^1$ may be selected from the group consisting of H, phenyl, biphenyl, pyridyl, and dibenzofuranyl.

$R^1$ may be selected from the group consisting of H and phenyl. $R^1$ may be H. $R^1$ may be phenyl.

$R^2$ and $R^3$ are independently $C_6$ to $C_{12}$, and at least one of $R^2$ and $R^3$ is phenyl. $R^1$ and $R^3$ may be independently selected from the group consisting of phenyl, biphenyl, and naphthyl, wherein at least one of $R^2$ and $R^3$ is phenyl. $R^2$ and $R^3$ may be independently selected from the group consisting of phenyl, biphenyl, wherein at least one of $R^2$ and $R^3$ is phenyl. $R^2$ is phenyl and $R^3$ may be phenyl; or $R^2$ is phenyl and $R^3$ is biphenyl.

In this regard, it may be provided that biphenyl is

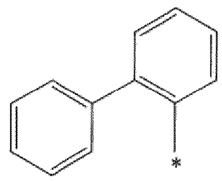

wherein "*" represents the binding position.

It may be provided that $R^2$ is phenyl and $R^3$ is phenyl.

The compound of formula (I) may be unsubstituted or substituted with one or more substituents independently selected from the group consisting of D, $C_1$ to $C_6$ alkyl; $C_1$ to $C_6$ alkoxy; and $SiR'_3$, wherein R' is independently selected from $C_1$ to $C_6$ alkyl.

Especially, if not mentioned explicitly elsewhere, all compounds, especially the compound of formula (I), groups, moieties, substituents etc. shown herein, especially by structural formulas, by systematic names etc. encompass the respective partially and fully deuterated derivatives thereof, wherein it may be provided that substitution beyond deuteration is excluded. If a compound, group, moiety, substituent etc. is described as unsubstituted, this does not exclude substitution with D.

The respective one or more substituents may be attached to any position of the structure of formula (I) by replacing a terminal hydrogen atom, including any respective position in L. Especially, all structural moieties and fragments of the compound of formula (I) shown herein, e.g. preferred structures of L, as described herein may be unsubstituted or substituted with the same substituents.

The compound of formula (I) may have a LUMO from -2.10 eV to -1.70 eV, preferred -2.05 eV to -1.80 eV, further preferred -2.01 eV to -1.84 eV, most preferred -2.00 eV to -1.91 eV.

The compound of formula (I) may have a glass transition temperature ≥ 90°C preferably ≥ 95°C, further preferred ≥ 99°C.

The compound of formula (I) may have a glass transition temperature from 90°C to 140°C, from 95°C to 120°C, or from 99°C to 114°C.

The compound of formula (I) may have a rate onset temperature from 200°C to 379°C, preferably from 200°C to 310°C

The compound of formula (I) may be selected from ET-1 to ET-22

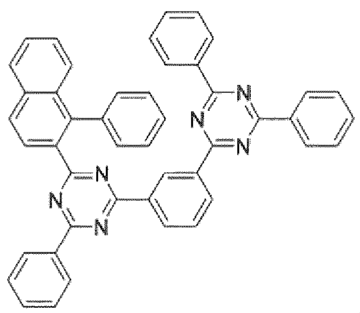

ET-1

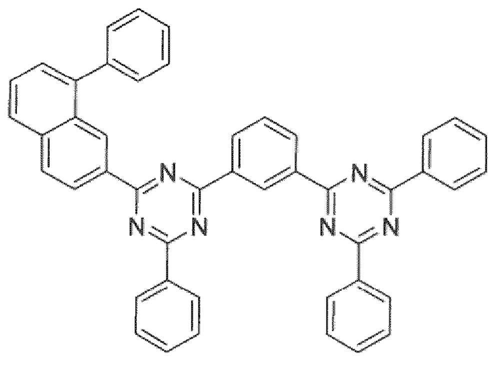

ET-2

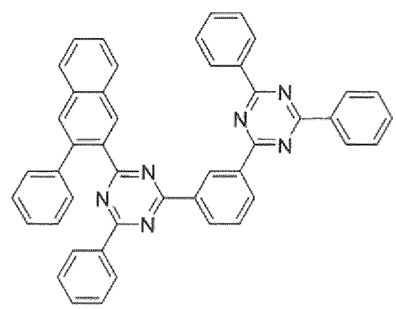

ET-3

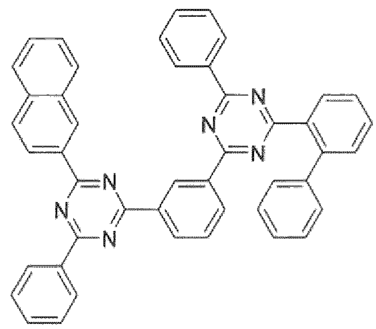

ET-4

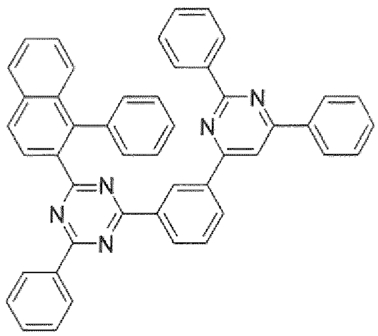
ET-5

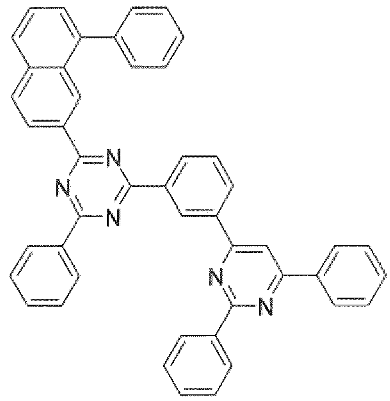
ET-6

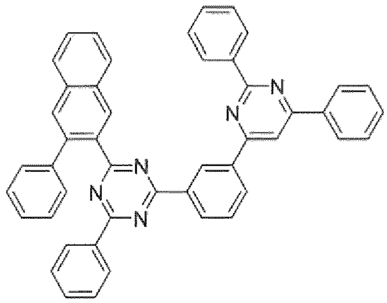
ET-7

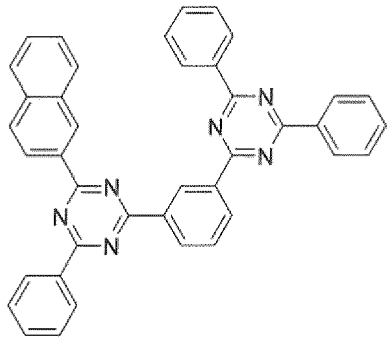
ET-8

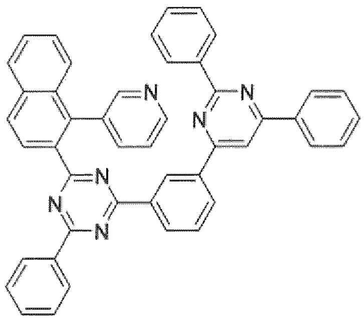
ET-9

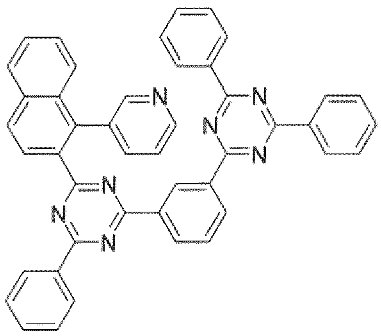
ET-10

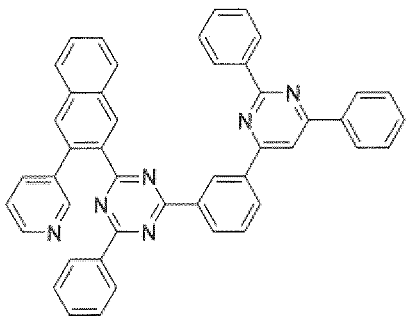
ET-11

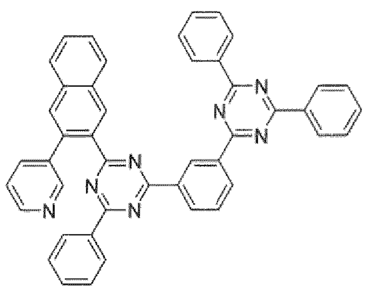
ET-12

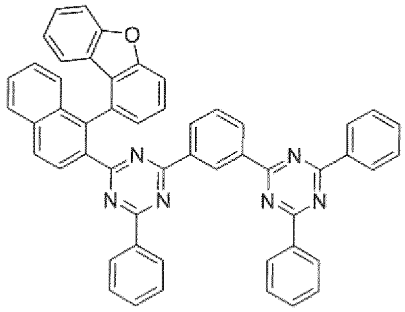

ET-13

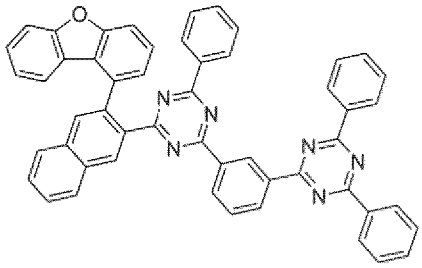

ET-14

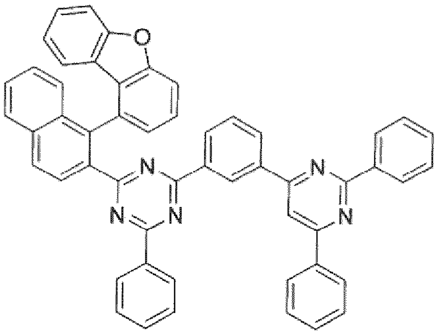

ET-15

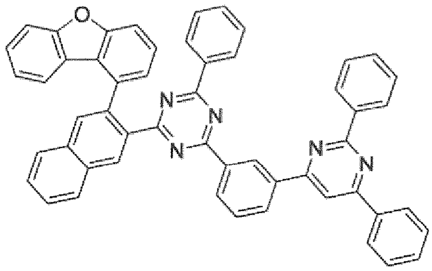

ET-16

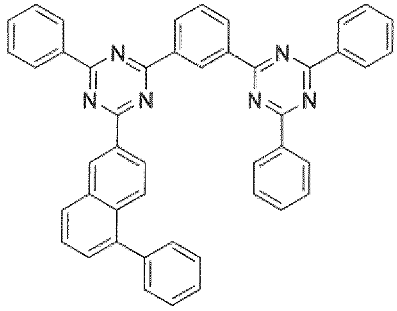

ET-17

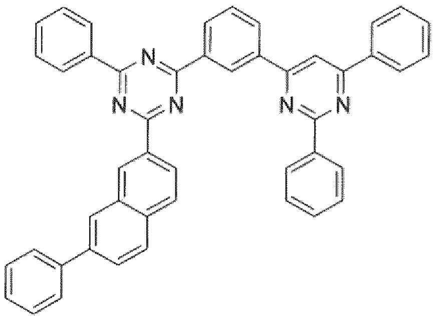

ET-18

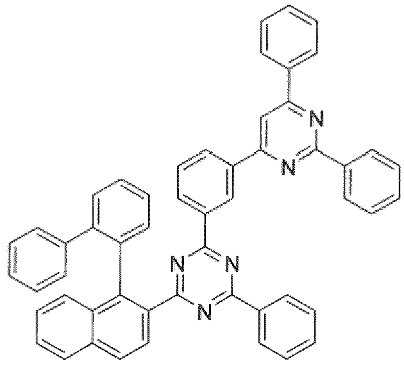

ET-19

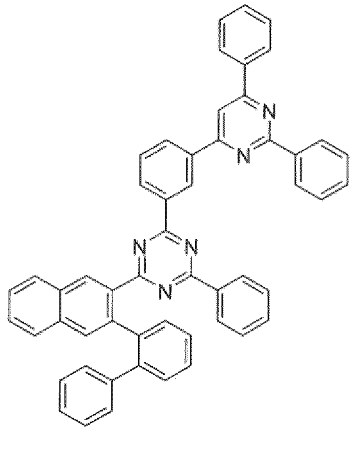

ET-20

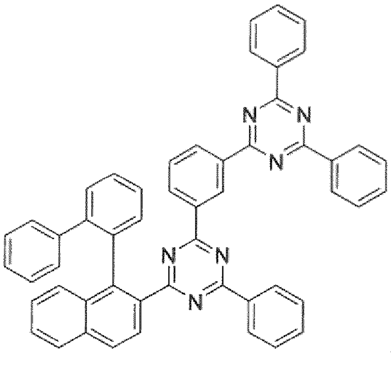

ET-21

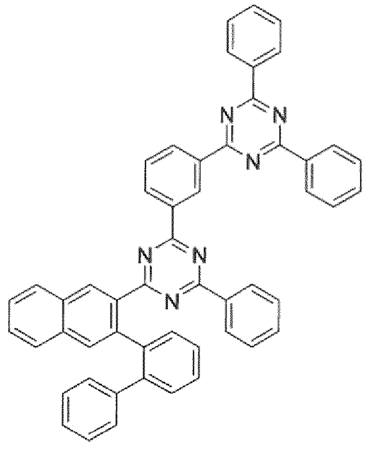

ET-22.

Organic light emitting diode

The organic light emitting diode according to the present invention comprises an anode, a cathode and an organic semiconducting layer. The organic semiconducting layer is arranged between the anode and the cathode. The organic semiconducting layer comprises the compound of formula (I) in accordance with the present invention, especially the compound as characterized above in detail.

The organic semiconducting layer comprises the compound of Formula (I). The organic semiconducting layer may comprise the compound of formula (I) in accordance with the invention in an amount of at least 30 wt.-% with respect to the total weight of the organic semiconducting layer. The organic semiconducting layer may comprise the compound of formula (I) in accordance with the invention in an amount of at least 40 wt.-% with respect to the total weight of the organic semiconducting layer. The organic semiconducting layer may comprise the compound of formula (I) in accordance with the invention in an amount of at least 50 wt.-% with respect to the total weight of the organic semiconducting layer. The organic semiconducting layer may comprise the compound of formula (I) in accordance with the invention in an amount of at least 60 wt.-% with respect to the total weight of the organic semiconducting layer. The organic semiconducting layer may comprise the compound of formula (I) in accordance with the invention in an amount of at least 70 w-t.-% with respect to the total weight of the organic semiconducting layer. The organic semiconducting layer may comprise the compound of formula (I) in accordance with the invention in an amount of at least 80 wt.-% with respect to the total weight of the organic semiconducting layer. The organic semiconducting layer may comprise the compound of formula (I) in accordance with the invention in an amount of at least 90 wt.-% with respect to the total weight of the organic semiconducting layer. The organic semiconducting layer may comprise the compound of formula (I) in accordance with the invention in an amount of at least 95 wt.-% with respect to the total weight of the organic semiconducting layer. The organic semiconducting layer may comprise the compound of formula (I) in accordance with the invention in an amount of at least 98 wt.-% with respect to the total weight of the organic semiconducting layer. The organic semiconducting layer may comprise the compound of formula (I) in accordance with the invention in an amount of at least 99 wt.-% with respect to the total weight of the organic semiconducting layer.

The organic semiconducting layer may consist of the compound of formula (I). Alternatively, the organic semiconducting layer may further comprise an electrical dopant, preferably an electrical n-dopant.

If the organic semiconducting layer consists of the compound of formula (I), that is, the organic semiconducting layer is undoped, it is preferred that $R^1$ bound to the following positions labeled with "*"

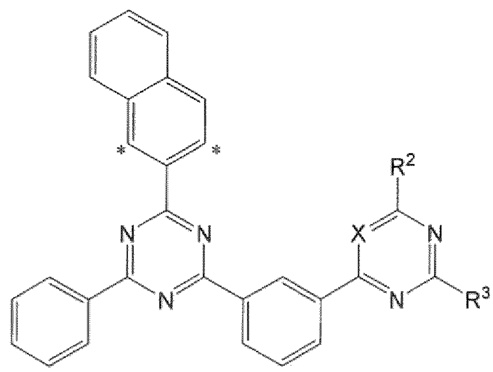

or that $R^1$ bound to the following position labeled with "*"

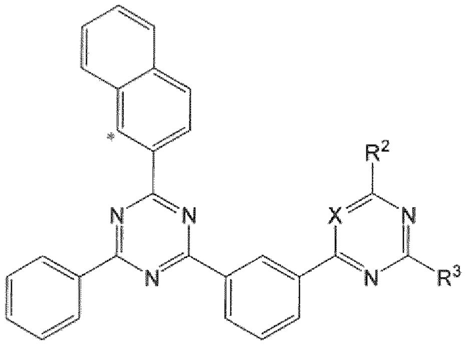

Under electrical n-dopant, it is understood a compound which, if embedded into an electron transport matrix, improves, in comparison with the neat matrix under the same physical conditions, the electron properties of the formed organic material, particularly in terms of electron injection and/or electron conductivity.

In the context of the present invention "embedded into an electron transport matrix" means homogenously mixed with the electron transport matrix.

The electrical n-dopant as referred to herein is especially selected from elemental metals, metal salts, metal complexes and organic radicals.

In one embodiment, the electrical n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

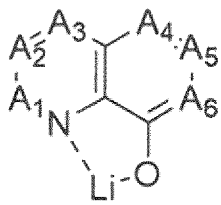 (II) 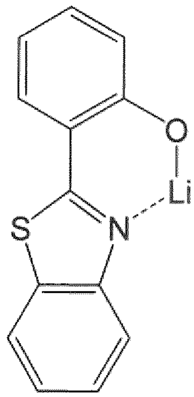 (III) 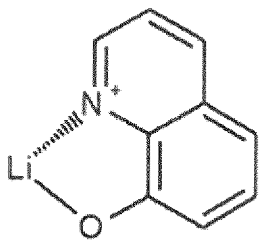 (IV)

  wherein

  $A_1$ to $A_6$ are same or independently selected from CH, CR, N, O;
  R is same or independently selected from hydrogen, halogen, alkyl or aryl or heteroaryl with 1 to 20 carbon atoms; and more preferred $A_1$ to $A_6$ are CH,

- the borate-based organic ligand is a tetra(1H-pyrazol-1-yl)borate,

- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,

- the pyridinolate is a 2-(diphenylphosphoryl)pyridin-3-olate,

- the lithium Schiff base has the structure 100, 101, 102 or 103:

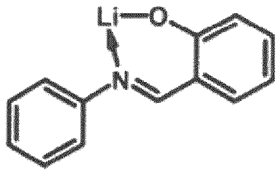
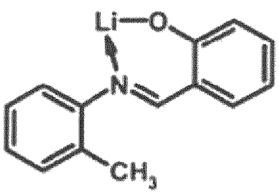
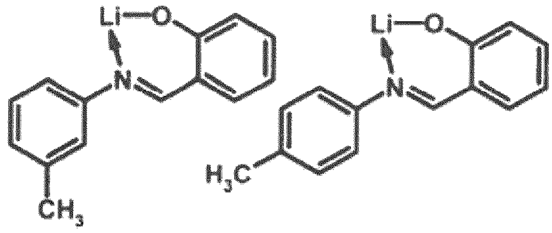

100 101 102 103

According to one embodiment of the invention, the organic semiconducting layer of the present invention comprises a lithium organic complex, and especially may comprise 8-hydroxyquinolinolato-lithium (= LiQ).

According to one embodiment of the present invention the organic semiconducting layer comprises a metal, preferably selected from alkali metals, alkaline earth metals, rare earth metals and metals of the transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The most practical benchmark for the strength of an electrical n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about -0.8 V to about -3.1 V; the practically applicable range of redox potentials for n-type dopants which can effectively n-dope such matrices is in a slightly broader range, from about -0.5 to about -3.3 V.

The measurement of redox potentials is practically performed for a corresponding redox couple consisting of the reduced and of the oxidized form of the same compound.

In case that the electrical n-type dopant is an electrically neutral metal complex and/or an electrically neutral organic radical, the measurement of its redox potential is actually performed for the redox couple formed by

(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than -0.5 V, preferably more negative than -1.2 V, more preferably more negative than -1.7 V, even more preferably more negative than -2.1 V, most preferably more negative than -2.5 V, if measured by cyclic voltammetry against the ferrocene/ferrocenium reference redox couple for a corresponding redox couple consisting of

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

In a preferred embodiment, the redox potential of the electrical n-dopant is between the value which is about 0.5 V more positive and the value which is about 0.5 V more negative than the value of the reduction potential of the chosen electron transport matrix.

Electrically neutral metal complexes suitable as electrical n-type dopants may be e.g. strongly reductive complexes of some transition metals in low oxidation state. Particularly strong electrical n-dopants may be selected for example from Cr(II), Mo(II) and/or W(II) guanidinate complexes such as $W_2(hpp)_4$, as described in more detail in WO2005/086251.

Electrically neutral organic radicals suitable as electrical n-dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form. It is further understood that if the vaporized elemental metal is deposited together with a covalent matrix, the metal atoms and/or clusters are embedded in the covalent matrix. In other words, it is understood that any metal-doped covalent material prepared by

vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the electrical n-dopant may be selected from electropositive metals selected from alkali metals, alkaline earth metals, rare earth metals and metals of the first transition period Ti, V, Cr and Mn. Preferably, the electrical n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The organic semiconducting layer may be an electron transport layer.

The organic light emitting diode may further comprise a light emitting layer, wherein the organic semiconducting layer is arranged between the light emitting layer and the cathode.

The organic light emitting diode may further comprise a light emitting layer, wherein the organic semiconducting layer is arranged between the light emitting layer and the cathode and the organic semiconducting layer is in direct contact with the light emitting layer.

The organic light emitting diode may further comprise a light emitting layer, wherein the organic semiconducting layer is arranged between the light emitting layer and the cathode, and the organic light emitting diode may further comprise an electron injection layer, and the organic semiconducting layer is arranged between the light emitting layer and the electron injection layer.

The organic light emitting diode may further comprise a light emitting layer, wherein the organic semiconducting layer is arranged between the light emitting layer and the cathode, and the organic light emitting diode further comprises an electron injection layer, and the organic semiconducting layer is arranged between and in direct contact with both the light emitting layer and the electron injection layer.

The organic light emitting diode may further comprise a light emitting layer, wherein the organic semiconducting layer is arranged between the light emitting layer and the cathode, and the organic light emitting diode may further comprise an auxiliary electron transport layer (= hole blocking layer), wherein the auxiliary electron transport layer is arranged between the light emitting layer and the organic semiconducting layer.

The organic light emitting diode may further comprise a light emitting layer, wherein the organic semiconducting layer is arranged between the light emitting layer and the cathode, and the organic light emitting diode may further comprise an auxiliary electron transport layer (= hole blocking layer), wherein the auxiliary electron transport layer is arranged between and in direct contact with both the light emitting layer and the organic semiconducting layer.

The organic light emitting diode may further comprise a light emitting layer, wherein the organic semiconducting layer is arranged between the light emitting layer and the cathode, and the organic light emitting diode may further comprise an auxiliary electron transport layer (= hole blocking layer), wherein the auxiliary electron transport layer is arranged between the light emitting layer and the organic semiconducting layer; and the organic light emitting diode further comprises an electron injection layer, and the organic semiconducting layer is arranged between the auxiliary electron transport layer and the electron injection layer.

The organic light emitting diode may further comprise a light emitting layer, wherein the organic semiconducting layer is arranged between the light emitting layer and the cathode, and the organic light emitting diode may further comprise an auxiliary electron transport layer (= hole blocking layer), wherein the auxiliary electron transport layer is arranged between and in direct contact with both the light emitting layer and the organic semiconducting layer; and the organic light emitting diode further comprises an electron injection layer, and the organic semiconducting layer is arranged between and in direct contact with both the auxiliary electron transport layer and the electron injection layer.

The organic light emitting diode may further comprise a light emitting layer, wherein the organic semiconducting layer is arranged between the light emitting layer and the cathode, and the organic light emitting diode may further comprise an auxiliary electron transport layer (= hole blocking layer), wherein the auxiliary electron transport layer is arranged between the light emitting layer and the organic semiconducting layer; and the organic light emitting diode further comprises an additional electron transport layer, and the organic semiconducting layer is arranged between the auxiliary electron transport layer and the additional electron transport layer.

The organic light emitting diode may further comprise a light emitting layer, wherein the organic semiconducting layer is arranged between the light emitting layer and the cathode, and the organic light emitting diode may further comprise an auxiliary electron transport layer (= hole blocking layer), wherein the auxiliary electron transport layer is arranged between and in direct contact with both the light emitting layer and the organic semiconducting layer; and the organic light emitting diode further comprises an additional electron transport layer, and the organic semiconducting layer is arranged between and in direct contact with both the auxiliary electron transport layer and the additional electron transport layer.

*Further layers*

In accordance with the invention, the organic light emitting diode may comprise, besides the layers already mentioned above, further layers. Exemplary embodiments of respective layers are described in the following:

*Substrate*

The substrate may be any substrate that is commonly used in manufacturing of electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate. It may be provided that the substrate is a non-transparent substrate.

*Anode electrode*

The organic light emitting diode comprises an anode electrode ("anode"). The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide ($SnO_2$), aluminum zinc oxide (AlZQ) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100 °C to 500 °C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80 °C to about 200 °C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of a p-type dopant and the p-type dopant may be selected from tetrafluoro-tetracyanoquinonedimethane ($F_4TCNQ$), 2,2-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), whose HOMO level is approximately -5.2 eV, doped with tetrafluoro-tetracyanoquinonedimethane ($F_4TCNQ$), whose LUMO level is about -5.2 eV; zinc phthalocyanine (ZnPc) (HOMO = -5.2 eV) doped with $F_4TCNQ$; $\alpha$-NPD (N,N'-Bis(naphthalen-1-yl)-N,N'-bis(phenyl)-benzidine) doped with $F_4TCNQ$; or $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. If the HTL is formed by vacuum deposition or spin coating, the conditions for deposition and coating may be similar to those for the formation of the HIL. However, the conditions for

the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The first emission layer and the second emission layer in the organic light emitting diode in accordance with the invention may be a blue emission layer or a green emission layer, respectively.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (I) and/or the compound of Formula (III) and/or the heterocyclic compound nCG.

The emission layer (EML) may be formed of a combination of a host and an emitter dopant. Example of the host are $Alq_3$, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc ($Zn(BTZ)_2$).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, $Ir(piq)_3$, and $Btp_2Ir(acac)$, but are not limited thereto. These compounds are phosphorescent emitters, however, fluorescent red emitter dopants could also be used.

Examples of phosphorescent green emitter dopants are $Ir(ppy)_3$ (ppy = phenylpyridine), $Ir(ppy)_2(acac)$, $Ir(mpyp)_3$.

Examples of phosphorescent blue emitter dopants are $F_2Irpic$, $(F_2ppy)_2Ir(tmd)$ and $Ir(dfppz)_3$ and ter-fluorene. 4.4'-bis(4-diphenyl amiostyryl)biphenyl (DPAVBi) and 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is

within this range, the EML may have excellent light emission, without a substantial penalty in driving voltage.

*Hole blocking layer (HBL)*

The OLED according to the present invention may comprise an auxiliary electron transport layer, also called hole blocking layer (HBL).

Especially if the organic light emitting device is a tandem OLED or another device comprising multiple layer stacks, the organic light emitting device may comprise more than one auxiliary electron transport layer ("hole blocking layer"), that is, one or more additional auxiliary electron transport layer(s).

Any compound that is commonly used to form a HBL may be used. Examples of compounds for forming the HBL include oxadiazole derivatives, triazole derivatives, and phenanthroline derivatives.

The HBL may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may have a thickness in the range from about 5 nm to about 100 nm, for example, from about 5 nm to about 30 nm. When the thickness of the HBL is within this range, the HBL may have excellent hole-blocking properties, without a substantial penalty in driving voltage.

In a preferred embodiment the HBL does not comprise a compound of formula (I).

*Electron transport layer (ETL)*

The OLED according to the present invention may comprise an electron transport layer (ETL). The electron transport layer may be the organic semiconducting layer comprising the compound of formula (I) or consisting of the compound of formula (I). If the OLED comprises more than one ETL, one or more of the ETLs may be the organic semiconducting layer comprising the compound of formula (I) or consisting of the compound of formula (I).

In an embodiment the ETL comprising the compound of formula (I) further comprises an electrical n-dopant.

Especially, if the organic light emitting device is a tandem OLED, the organic light emitting device may comprise more than one electron transport layer, that is, one or more additional electron transport layer(s) ("additional ETL").

In one embodiment, the electron transport matrix compounds in the additional ETLs consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

*Electron injection layer (EIL)*

An EIL, which may facilitate injection of electrons from the cathode into the electron transport layer stack, may be formed on the electron transport layer, preferably directly on the electron transport layer.

It may be provided that the electron injection layer does not comprise the compound of formula (I).

The electron injection layer may comprise a metal salt or a metal complex, preferably a lithium salt or a lithium organic complex; more preferably a compound selected from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

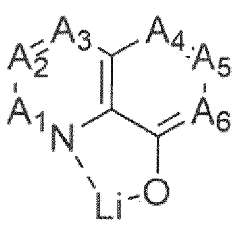 (II), 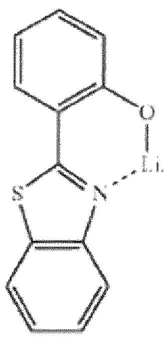 (III), 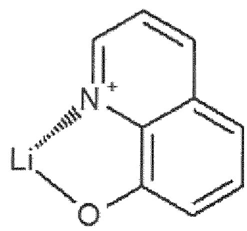 (IV)

wherein

$A_1$ to $A_6$, are same or independently selected from CH, CR, N, O;

R is same or independently selected from hydrogen, halogen, alkyl or aryl or heteroaryl with 1 to 20 carbon atoms; and more preferred $A_1$ to $A_6$ are CH,

- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

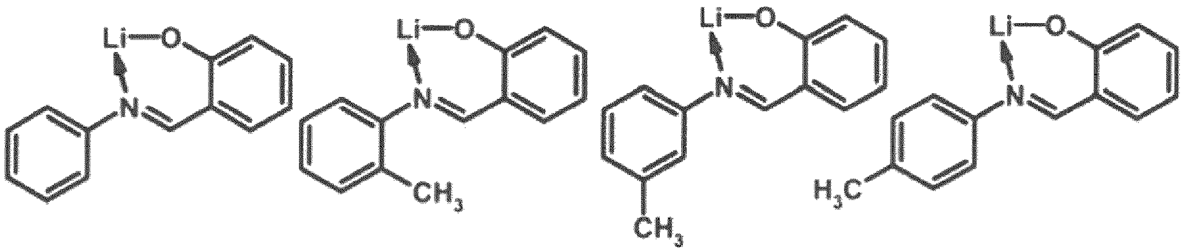

100 101 102 103

The electron injection layer may comprise or consist of a metal selected from the group consisting of alkali metals, alkaline earth metals, and rare earth metals, preferably the metal may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, more preferably from Li, Na and Yb, most preferably Yb.

The thickness of the electron injection layer may be in the range from about 0.1 nm to about 10 nm, for example, in the range from about 0.5 nm to about 5 nm. When the thickness of the EIL is within this range, the EIL may have satisfactory electron-injecting properties, without a substantial penalty in driving voltage.

*Cathode electrode*

The cathode (cathode electrode) is formed on the EIL (if present) or on the ETL, preferably directly on the EIL, preferably in direct contact with the EIL. In the sense of this invention the cathode and the EIL can be regarded as one functional part enabling the injection of electrons into the electron transport layer stack. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like, for example an alloy of Ag and Mg, such as Ag:Mg 90:10 wt/wt. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. If the thickness of the cathode electrode is in the range from about 5 nm to about 50 nm, the cathode electrode may be transparent or semitransparent even if formed from a metal or metal alloy. The transparent or semitransparent cathode may facilitate light emission through the cathode.

*Charge generation layer (CGL)*

The charge generation layer (CGL) may comprise a p-type charge generation layer (p-CGL) and an n-type charge generation layer (n-CGL). An interlayer may be arranged between the p-CGL and the n-CGL.

Typically, the charge generation layer is a p-n junction joining an n-type charge generation layer (electron generating layer) and a hole generating layer. The n-side of the p-n junction generates electrons and injects them into the layer that is adjacent in the direction to the anode. Analogously, the p-side of the p-n junction generates holes and injects them into the layer that is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode,

while the hole generating layer provides holes to the second light emission layer arranged between the first emission layer and the cathode.

Suitable matrix materials for the hole generating layer may be materials conventionally used as hole injection and/or hole transport matrix materials. Also, the p-type dopant used for the hole generating layer can consist of conventional materials. For example, the p-type dopant can be one selected from a group consisting of tetrafluoro-7,7,8,8-tetracyanoquinodimethane ($F_4$-TCNQ), derivatives of tetracyanoquinodimethane, radialene derivatives, iodine, $FeCl_3$, $FeF_3$, and $SbCl_5$. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline) aluminum, benzazole derivatives and silole derivatives.

According to one aspect of the present invention, an electron transport layer comprising the compound of formula (I) is arranged between a first and a second emission layer and a further electron transport layer comprising the compound of formula (I) is arranged between the second emission layer and the cathode.

According to another aspect of the present invention, there is provided an OLED comprising: a substrate; an anode electrode formed on the substrate; a hole injection layer, a hole transport layer, an electron blocking layer, an emission layer, an auxiliary electron transport layer, an electron transport layer comprising the compound of formula (I) and a cathode electrode.

According to another aspect of the present invention, there is provided an OLED comprising: a substrate; an anode electrode formed on the substrate; a hole injection layer, a hole transport layer, an electron blocking layer, an emission layer, an auxiliary electron transport layer, an electron transport layer comprising the compound of formula (I), an electron injection layer, and a cathode electrode.

According to various embodiments of the present invention, there may be provided further layers conventionally used in OLEDs layers arranged between the above mentioned layers, on the substrate or on the top electrode.

In one embodiment, the organic light emitting device according to the invention further comprises a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluoromethyl, perfluoroethyl, perfluoropropyl, perfluoroisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl and the like.

In one embodimentthe hole injection, hole transporting and/or a hole generation layer may comprise the radialene and/or the quinodimethane compound.

In one embodiment, the radialene compound may have Formula (XX) and/or the quinodimethane compound may have Formula (XXIa) or (XXIb):

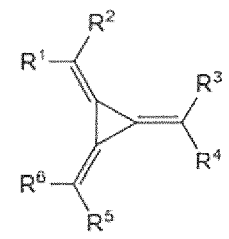 (XX) 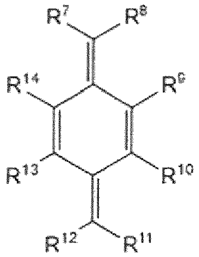 (XXIa) 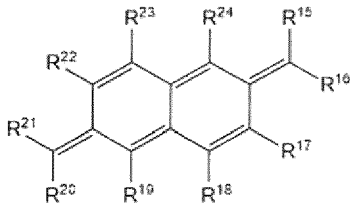 (XXIb),

wherein (as an exception different to the description above) $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

Process for preparing the organic light emitting device

According to a further aspect, the invention is related to a process for preparing the organic light emitting device according to the present invention, wherein the process comprises a step of depositing the compound of formula (I) according to the present invention on a solid support.

The method for depositing may comprise:

- deposition via vacuum thermal evaporation;
- deposition via solution processing, preferably the processing is selected from spin-coating, printing, casting; and/or
- slot-die coating.

Device

According to a further aspect, the invention is related to a display device or a lighting device comprising the organic light emitting device according to the invention, preferably comprising at least two organic light emitting devices according to the invention.

The display device may be a television, a tablet, or a mobile phone.

GENERAL DEFINITIONS

If not explicitly mentioned else, each moiety of the compounds described herein, especially the compounds of formula (I) may be substituted with one or more D (deuterium).

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl groups. For example, $C_3$-alkyl may be selected from n-propyl and iso-propyl. Likewise, $C_4$-alkyl encompasses n-butyl, sec-butyl, iso-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The term "aryl" or "arylene" as used herein shall encompass phenyl ($C_6$-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzene groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrenyl group, a fluorenyl group and the like. Further encompoassed are spiro compounds in which two aromatic moieties are connected to each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom. The heteroatom may be selected from N, O, S, B, Si, P, Se, preferably from N, O and S. A heteroarylene ring may comprise at least 1 to 3

heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1 = CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazole, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In accordance with the present disclosure, as an illustrative example using an arbitrary group A in a formula showing the following binding situation,

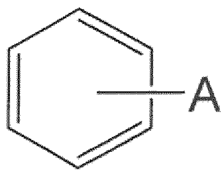

the group A may be bound to any suitable binding position. In a situation where it is shown that the bond of A crosses more than one ring

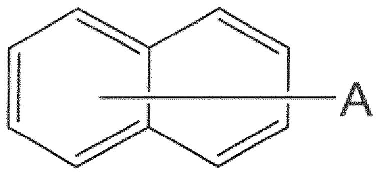

the group A may be bound to any suitable binding position of each ring crossed with the bond.

In terms of the invention, the expression "between" with respect to one layer being between two other layers does not exclude the presence of further layers, which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light through a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in its structural formula.

The terms "OLED" and "organic light-emitting diode" are used simultaneously and have the same meaning. The term "organic electroluminescent device" as used herein may comprise both organic light emitting diodes as well as organic light-emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is understood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about", the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the electron transport layer comprising the compound of Formula (I) is essentially non-emissive or non-emitting.

The operating voltage, also named U, is measured in Volt (V) at 10 milliampere per square centimeter ($mA/cm^2$).

The candela per ampere efficiency, also named cd/A, efficiency or $C_{eff}$, is measured in candela per ampere at 10 milliampere per square centimeter ($mA/cm^2$).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

Hereinafter, the embodiments are illustrated in more detail with reference to examples. However, the present disclosure is not limited to the following examples. Reference will now be made in detail to the exemplary aspects.

## DESCRIPTION OF THE FIGURES

The aforementioned components, as well as the claimed components and the components to be used in

accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures, which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an OLED, according to an exemplary embodiment of the present invention.

FIG. 2 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, and an electron transport layer (ETL) 160. The ETL 160 comprises the compound of formula (I). The electron transport layer (ETL) 160 is formed on the HBL 155. Onto the electron transport layer (ETL) 160, an electron injection layer (EIL) 180 is disposed. The cathode 190 is disposed directly onto the electron injection layer (EIL) 180.

Fig. 2 is a schematic sectional view of an OLED 100, according to another exemplary embodiment of the present invention. Fig. 2 differs from Fig. 1 in that the OLED 100 of Fig. 2 further comprises a charge generation layer (CGL) and a second emission layer (151).

Referring to Fig. 2, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (HBL) 156, a second electron transport layer (ETL) 161 which is the electron transport layer comprising the compound of formula (I), a second electron injection layer (EIL) 181 and a cathode 190.

## EXPERIMENTAL PART

### Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 $\mu$L Mettler Toledo aluminum pan with lid, a <1 mm hole is pierced into the lid).

### Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

### Rate onset temperature

The rate onset temperature (TRO) is determined by loading 100 mg compound into a VTE source. As VTE source a point source for organic materials may be used as supplied by Kurt J. Lesker Company (www.lesker.com) or CreaPhys GmbH (http://www..creaphys.com). The VTE source is heated at a constant rate of 15 K/min at a pressure of less than 10-5 mbar and the temperature inside the source measured with a thermocouple. Evaporation of the compound is detected with

a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 oC. If the rate onset temperature is below 200 oC the evaporation may be too rapid and therefore difficult to control. If the rate onset temperature is above 255 oC the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with potentioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc+/Fc redox couple, afforded finally the values reported above. All studied compounds as well as the reported comparative compounds showed well-defined reversible electrochemical behaviour.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). If more than one conformation is viable, the conformation with the lowest total energy is selected to determine the bond lengths of the molecules.

Calculated HOMO, LUMO, T1 Triplet level

The HOMO, LUMO and T1 are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The dipole moment, the HOMO and LUMO energy levels of the molecular structures are determined by applying the hybrid functional B3LYP with a 6-31G* basis set from the optimized geometries obtained by applying the hybrid functional B3LYP with a 6-31G* basis set in the gas phase. All the calculations were performed in the gas phase. If more than one conformation is viable, the conformation with the lowest total energy is selected.

Synthesis Procedures

*Synthesis of ET- 1*

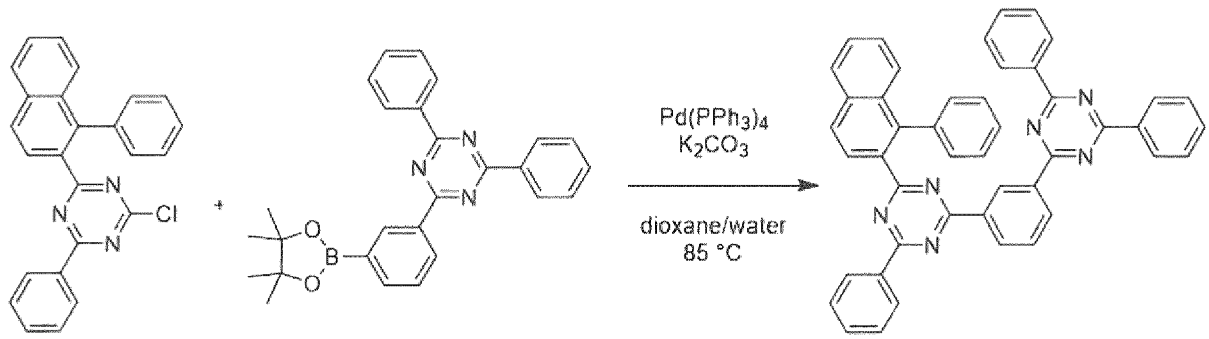


2-chloro-4-phenyl-6-(1-phenylnaphthalen-2-yl)-1,3,5-triazine (20.0 g, 50.8 mmol), 2,4-diphenyl-6-(3-(4,4,5,5-tetramethyl-1,3,2-dioxaborolan-2-yl)phenyl)-1,3,5-triazine (22.1 g, 50.8 mmol) and potassium carbonate (14.0 g, 102 mmol) were mixed under a nitrogen atmosphere and dissolved in 1,4-dioxane (306 ml) and water (51 ml). Tetrakis(triphenylphosphine)palladium(o) (1.17 g, 1.02 mmol) was then added and the mixture heated at reflux for 16 h. After cooling to RT, the resulting grey precipitate was collected by filtration and washed with water, hexane and methanol. This crude product was extracted continuously into toluene (700 ml) using a Soxhlet apparatus. Cooling the resulting mixture gave a colourless precipitate, which was collected by filtration, washed with hexane and dried under vacuum to yield the product (30.8 g, 91%).

ESI-MS: m/z = 667 ($[MH]^+$)

*Synthesis of ET-2*

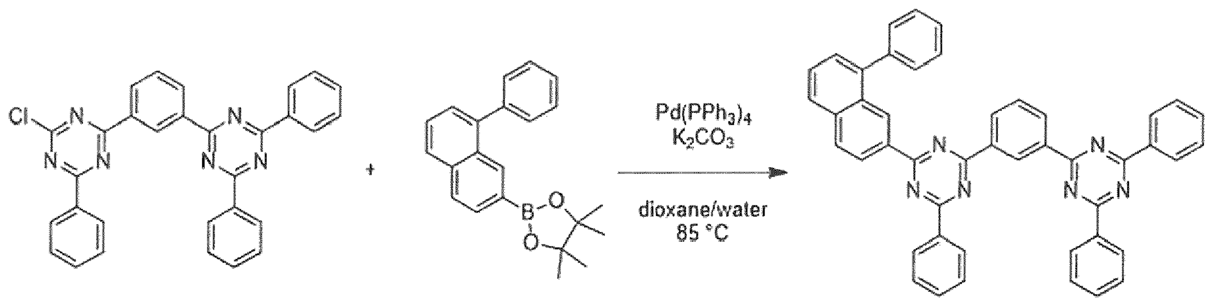


2-chloro-4-(3-(4,6-diphenyl-1,3,5-triazin-$_2$-yl)phenyl)-6-phenyl-1,3,5-triazine (10.0 g, 20.0 mmol), 4,4,5,5-tetramethyl-2-(8-phenylnaphthalen-2-yl)-1,3,2-dioxaborolane (7.28 g, 22.0 mmol) and potassium carbonate (6.93 g, 50.1 mmol) were mixed under a nitrogen atmosphere and dissolved in 1,4-dioxane (360 ml) and water (40 ml). Tetrakis(triphenylphosphine)palladium(0) (0 463 g, 0 401 mmol) was then added and the mixture heated at reflux for 16 h. Reaction control indicated incomplete conversion, so further portions of 4,4,5,5-tetramethyl-2-(8-phenylnaphthalen-2-yl)-1,3,2-dioxaborolane (3.64 g, 11.0 mmol) and tetrakis(triphenylphosphine)palladium(o) (o 231 g, 0 200 mmol) were added, and the mixture heated for a further 20 h at reflux. After cooling to RT, the resulting grey precipitate was collected by filtration and washed with water and methanol. This crude product was extracted continuously into toluene (700 ml) using a Soxhlet apparatus. Due to incomplete extraction, the residue was further extracted with chlorobenzene (500 ml). After removal of the respective solvents under vacuum, the fractions were combined and macerated with hot tetrahydrofuran. The resulting solid was collected by filtration and dried under vacuum to provide the product (9.4 g, 70%).

ESI-MS: m/z = 667 ($[MH]^+$)

*Synthesis of ET-3*

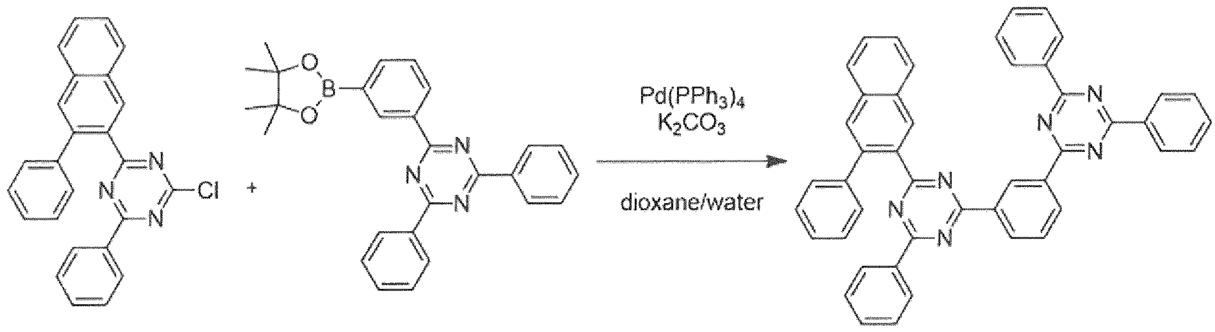


2-chloro-4-phenyl-6-(3-phenylnaphthalen-2-yl)-1,3,5-triazine (4.00 g, 10.2 mmol), 2,4-diphenyl-6-(3-(4,4,5,5-tetramethyl-1,3,2-dioxaborolan-2-yl)phenyl)-1,3,5-triazine (4.42 g, 10.2 mmol) and potassium carbonate (2.81 g, 20.3 mmol) were mixed under a nitrogen atmosphere and dissolved in 1,4-dioxane (90 ml) and water (10 ml). Tetrakis(triphenylphosphine)palladium(0) (0.235 g, 0.203 mmol) was then added and the mixture heated at reflux for 16 h. After cooling to RT, the resulting grey precipitate was collected by filtration and washed with water and methanol. This crude product was extracted continuously into toluene (300 ml) using a Soxhlet apparatus. Cooling the resulting mixture gave a colourless precipitate, which was collected by filtration, washed with hexane and acetone, and dried under vacuum to yield the product (5.37 g, 79%).

ESI-MS: m/z = 667 ([MH]+)

*Synthesis of ET-4*

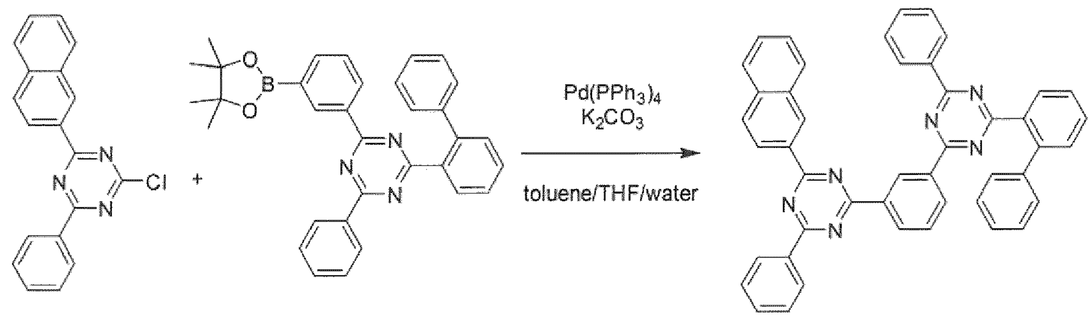


2-chloro-4-(naphthalen-2-yl)-6-phenyl-1,3,5-triazine (10.0 g, 31.5 mmol), 2-([1,1'-biphenyl]-2-yl)-4-phenyl-6-(3-(4,4,5,5-tetramethyl-1,3,2-dioxaborolan-2-yl)phenyl)-1,3,5-triazine (15.3 g, 29.9 mmol) and potassium carbonate (8.70 g, 62.9 mmol) were mixed under a nitrogen atmosphere and dissolved in a mixture of toluene (120 ml), THF (120 ml) and water (30 ml). Tetrakis(triphenylphosphine)palladium(o) (0.727 g, 0.629 mmol) was then added and the mixture heated at 60 °C for 16 h, then a further 20 h at 74 °C. After cooling to RT, the resulting pale precipitate was collected by filtration and washed with toluene, water and methanol. This crude product was extracted continuously into toluene (600 ml) using a Soxhlet apparatus. The volatiles were removed from the filtrate under reduced pressure, and the residue was triturated with hot THF and then acetonitrile, collected again by filtration and dried under vacuum to yield the product (17.4 g, 83%).

ESI-MS: m/z = 667 ([MH]+)

*Synthesis of ET-5*

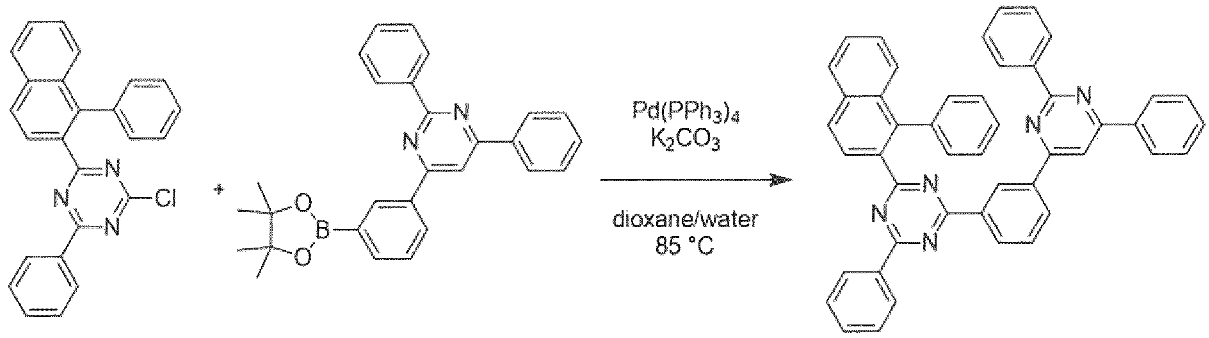


2-chloro-4-phenyl-6-(1-phenylnaphthalen-2-yl)-1,3,5-triazine (10.0 g, 25.4 mmol), 2,4-diphenyl-6-(3-(4,4,5,5-tetramethyl-1,3,2-dioxaborolan-2-yl)phenyl)pyrimidine (11.0 g, 25.4 mmol) and potassium carbonate (7.02 g, 51 mmol) were mixed under a nitrogen atmosphere and dissolved in 1,4-dioxane (150 ml) and water (25 ml). Tetrakis(triphenylphosphine) palladium(o) (o 587 g, o 51 mmol) was then added and the mixture heated at reflux for 16 h. After cooling to RT, the resulting grey precipitate was collected by filtration and washed with water and 1,4-dioxane. This crude product was extracted continuously into chlorobenzene (300 ml) using a Soxhlet apparatus. Cooling the resulting mixture gave a colourless precipitate, which was collected by filtration, washed with chloroform and hexane, and dried under vacuum to yield the product (14.7 g, 87%).

ESI-MS: m/z = 666 ($[MH]^+$)

*Synthesis of ET-6*

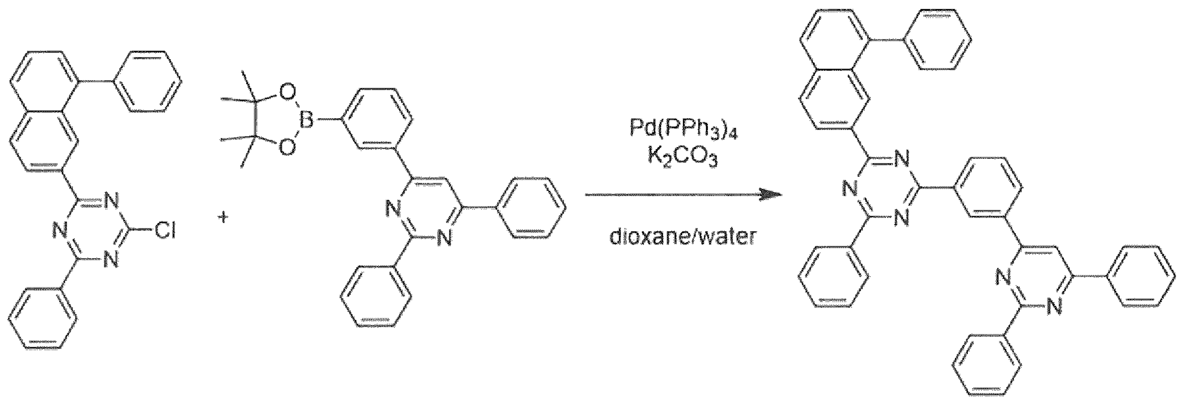


2-chloro-4-phenyl-6-(8-phenylnaphthalen-2-yl)-1,3,5-triazine (11.2 g, 28.4 mmol), 2,4-diphenyl-6-(3-(4,4,5,5-tetramethyl-1,3,2-dioxaborolan-2-yl)phenyl)pyrimidine (12.3 g, 28.4 mmol) and potassium carbonate (7.84 g, 56.7 mmol) were mixed under a nitrogen atmosphere and dissolved in 1,4-dioxane (240 ml) and water (30 ml). Tetrakis(triphenylphosphine)palladium(o) (o 655 g, 0 567 mmol) was then added and the mixture heated at reflux for 16 h. After cooling to RT, the resulting grey precipitate was collected by filtration and washed with water, methanol and hexane. This crude product was extracted continuously into chlorobenzene (700 ml) using a Soxhlet apparatus. Cooling the resulting mixture gave a colourless precipitate, which was collected by filtration, washed with hexane, and dried under vacuum to yield the product (16.9 g, 90%).

ESI-MS: m/z = 666 ($[MH]^+$)

*Synthesis of ET-7*

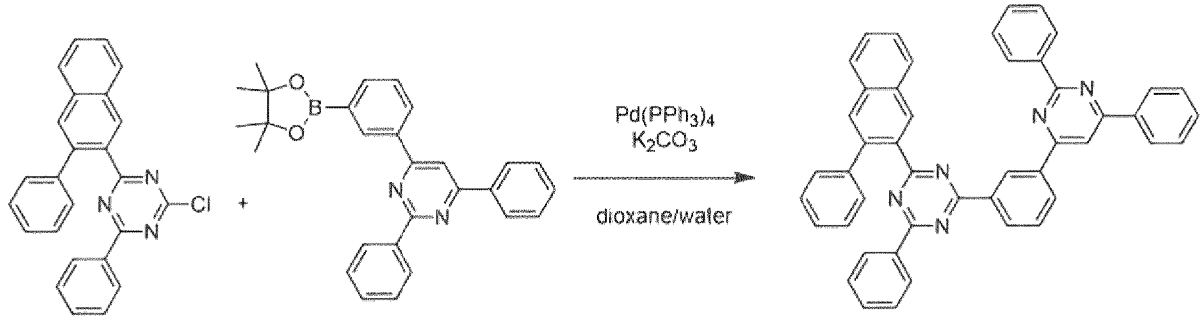


2-chloro-4-phenyl-6-(3-phenylnaphthalen-2-yl)-1,3,5-triazine (4.10 g, 10.4 mmol), 2,4-diphenyl-6-(3-(4,4,5,5-tetramethyl-1,3,2-dioxaborolan-2-yl)phenyl)pyrimidine (4.52 g, 10.4 mmol) and potassium carbonate (2.88 g, 20.8 mmol) were mixed under a nitrogen atmosphere and dissolved in 1,4-dioxane (110 ml) and water (11 ml). Tetrakis(triphenylphosphine)palladium(o) (0.241 g, 0.208 mmol) was then added and the mixture heated at reflux for 16 h. After cooling to RT, the resulting grey precipitate was collected by filtration and washed with water and methanol. The solid was then dissolved in dichloromethane (400 ml) and passed through a pad of silica gel. Reduction of the volume to 100 ml and addition of methanol (100 ml) gave a precipitate, which was collected by filtration and dried under vacuum to provide the product (6.60 g, 95%).

ESI-MS: m/z = 666 ($[MH]^+$)

*Synthesis of ET-8*

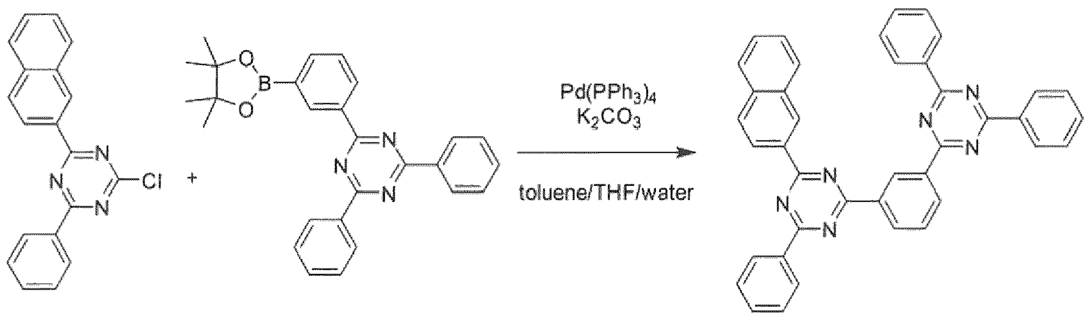


2-chloro-4-(naphthalen-2-yl)-6-phenyl-1,3,5-triazine (14.5 g, 45.6 mmol), 2,4-diphenyl-6-(3-(4,4,5,5-tetramethyl-1,3,2-dioxaborolan-2-yl)phenyl)-1,3,5-triazine (20.9 g, 47.9 mmol) and potassium carbonate (12.6 g, 91.2 mmol) were mixed under a nitrogen atmosphere and dissolved in a mixture of toluene (200 ml), THF (590 ml) and water (120 ml). Tetrakis(triphenylphosphine)palladium(o) (0.727 g, 0.629 mmol) was then added and the mixture heated at 75 °C for 19 h. After cooling to RT, the resulting pale precipitate was collected by filtration, washed with water and macerated with hot toluene. The solid was then dissolved in chlorobenzene and filtered through a pad of silica gel. Hexane was then added to precipitate the product, which was dried under vacuum (24.9 g, 93%).

Properties of inventive compounds

Table 1a:

| | ET-1 | ET-2 | ET-3 | ET-4 | ET-5 | ET-6 | ET-7 | ET-8 |
|---|---|---|---|---|---|---|---|---|
| LUMO [eV] | -1.95 | -2.00 | -1.95 | -1.98 | -1.91 | -2.00 | -1.91 | -1.99 |
| HOMO [eV] | -5.83 | -5.87 | -5.83 | -5.92 | -5.85 | -5.81 | 5.87 | -5.94 |
| T1 [eV] | 2.52 | 2.47 | 2.58 | 2.50 | 2.52 | 2.42 | 2.56 | 2.49 |
| Tg [°C] | 114 | 109 | 112 | 99 | 110 | 102 | 111 | 108 |

(continued)

| | ET-1 | ET-2 | ET-3 | ET-4 | ET-5 | ET-6 | ET-7 | ET-8 |
|---|---|---|---|---|---|---|---|---|
| Tm [°C] | 286 | 334 | 275 | not obs. | not obs. | 315 | not obs. | 270 |
| TRO Rate onset [°C] | 255 | 292 | 257 | 242 | 246 | 280 | 258 | 247 |

Table 1b:

| | ET-9 | ET-10 | ET-11 | ET-12 | ET-13 | ET-14 | ET-15 | ET-16 |
|---|---|---|---|---|---|---|---|---|
| LUMO [eV] | -1.94 | -1.95 | -1.96 | -1.98 | -1.93 | -1.95 | -1.93 | -1.88 |
| HOMO [eV] | -5.96 | -5.90 | -6.01 | -5.98 | -5.81 | -5.80 | -5.92 | -5.81 |
| T1 [eV] | 2.52 | 2.53 | 2.57 | 2.56 | 2.52 | 2.58 | 2.55 | 2.56 |

Table 1c:

| | ET-17 | ET-18 | ET-19 | ET-20 | ET-21 | ET-22 |
|---|---|---|---|---|---|---|
| LUMO [eV] | -1.98 | -1.97 | -1.84 | -1.87 | -1.93 | -1.94 |
| HOMO [eV] | -5.76 | -5.80 | -5.75 | -5.84 | -5.72 | -5.71 |
| T1 [eV] | 2.43 | 2.44 | 2.49 | 2.56 | 2.51 | 2.54 |

Device experiments

*General procedure for fabrication of OLEDs*

For the top emission OLED devices an ITO / Ag / ITO substrate with dimensions of 100 mm x 100 mm x 0.7 mm was ultrasonically cleaned with DI water rinse for 20 minutes, then with Isopropanol for 20 minutes in beaker, and dried for 15 minutes in a spin rinse dryer. Subsequently, the substrate was baked out in oven at 200°C for ~2h. Before organic material deposition, it was subjected to plasma treatment in vacuum chamber (typically $N_2$:$O_2$ plasma).

The device according to layer stack A was made by depositing a hole injection layer of HT-1 doped with D-1 onto a glass substrate provided with ITO / Ag / ITO anode, followed by an undoped hole transport layer of HT-1. Subsequently, an electron blocking layer of HT-2 was deposited onto the hole transport layer. Subsequently, a blue fluorescent emitting layer of emitter host Host-1 doped with Emitter-1 was deposited. Subsequently, a hole blocking layer of E1 was deposited on the emission layer. Subsequently, an electron transport layer made of the comparative compound C-1 or the inventive compounds was deposited onto the hole blocking layer. Subsequently, a first electron injection layer of E2 was co-deposited with Li onto the electron transport layer. Subsequently, a second electron injection layer made of Yb was deposited onto the first electron injection layer, followed by a cathode consisting of a silver-magnesium alloy in a volume ratio 90:10. Finally, a capping layer made of HT-1 was deposited on top of the cathode. All depositions were performed by vacuum thermal evaporation.

*Layer stack A for OLED devices with undoped ETL*

ITO / HT-1:D-1 (2 vol96), 10nm / HT-1, 122nm / HT-2, 5nm / HOST-1:EMITTER-1 (3 vol%), 20nm / E1, 5nm / f(I) or comparative compound, 20nm / E2:Li (1 vol%) 10nm / Yb, 2nm / Ag:Mg (90:10 vol%), 13 nm / HT-1, 75 nm

The device according to layer stack B was made by depositing a hole injection layer of HT-1 doped with D-1 onto a glass substrate provided with ITO / Ag / ITO anode, followed by an undoped hole transport layer of HT-1. Subsequently, an electron blocking layer of HT-2 was deposited onto the hole transport layer. Subsequently, a blue fluorescent emitting layer of emitter host Host-1 doped with Emitter-i was deposited. Subsequently, a hole blocking layer of E1 was deposited on the emission layer. Subsequently, an electron transport layer made of the comparative compound C-1 or the inventive compounds was co-deposited with LiQ (50 vol%) onto the hole blocking layer. Subsequently, an electron injection layer made of Yb was deposited onto the electron transport layer, followed by a cathode consisting of a silver-magnesium alloy in a volume ratio 90:10. Finally, a capping layer made of HT-1 was deposited on top of the cathode. All depositions were performed by vacuum thermal evaporation.

*Layer stack B for OLED devices with LiQ-doped ETL*

ITO / HT-1:D-1 (2 vol%), 10nm / HT-1, 122nm / HT-2, snm / HOST-1:EMITTER-1 (3 vol%), 20nm / E1, 5nm / f(I):LiQ or comparative compound:LiQ (50%), 31nm / Yb, 2nm / Ag:Mg (90:10 vol%), 13 nm / HT-1, 75 nm

| | IUPAC name | Reference |
|---|---|---|
| HT-1 | N-([1,1'-biphenyl]-4-yl)-9,9-dimethyl-N-(4-(9-phenyl-9H-carbazol-3-yl)phenyl)-9H-fluoren-2-amine [CAS 1242056-42-3] | US2016322581 |
| HT-2 | N-([1,1'-biphenyl]-4-yl)-9,9-diphenyl-N-(4-(triphenylsilyl) phenyl)-gH-fluoren-2-amine [CAS 1613079-70-1] | - |
| D-1 | 4,4',4"-((1E,1'E,1"E)-cyclopropane-1,2,3-triylidenetris(cyanomethanylylidene))tris(2,3,5,6-tetrafluorobenzonitrile) [CAS 1224447-88-4] | US2008265216 |
| HOST-1 | H09 | Commercially available from Sun Fine Chemicals, Inc, S. Korea |
| EMITTER-1 | BD200 | Commercially available from Sun Fine Chemicals, Inc, S. Korea |
| E1 | 2-(3'-(9,9-dimethyl-9H-fluoren-2-yl)-[1,1'-biphenyl]-3-yl)-4,6-diphenyl-1,3,5-triazine [CAS 1955543-57-3] | WO2016105141 |
| E2 | 2,2'-(1,3-Phenylene)bis[9-phenyl-1,10-phenanthroline] [CAS 721969-94-4] | JP2004281390 |
| LiQ | 8-Hydroxyquinolinolato-lithium [CAS 850918-68-2] | WO2013079217 |

*General procedure for measurement of OLEDs*

To assess the performance of the inventive examples compared to the prior art, the current efficiency is measured at 20 °C. The current-voltage characteristic is determined using a Keithley 2635 source measure unit, by sourcing a voltage in V and measuring the current in mA flowing through the device under test. The voltage applied to the device is varied in steps of 0.1 V in the range between 0 V and 10 V. Likewise, the luminance-voltage characteristic and CIEy, coordinates are determined by measuring the luminance in cd/$m^2$ using an Instrument Systems CAS-140CT array spectrometer (calibrated by Deutsche Akkreditierungsstelle (DAkkS)) for each of the voltage values. The cd/A efficiency at 10 mA/$cm^2$ is determined by interpolating the luminance-voltage and current-voltage characteristics, respectively.

In bottom emission devices, the emission is predominantly Lambertian and quantified in percent external quantum efficiency (EQE). To determine the efficiency EQE in %, the light output of the device is measured using a calibrated photodiode at 10 mA/$cm^2$.

In top emission devices, the emission is forward directed, non-Lambertian and also highly dependent on the micro-cavity. Therefore, the efficiency EQE is higher compared to bottom emission devices. To determine the efficiency EQE in % the light output of the device is measured using a calibrated photodiode at 10 mA/$cm^2$,

<u>Tested compounds</u>

*Comparative compound*

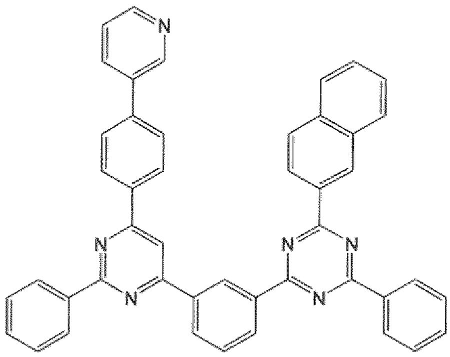

C-1

*Compounds according to the invention*

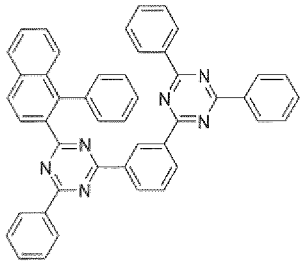

ET-1

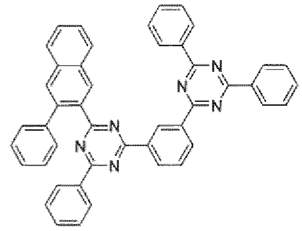

ET-3

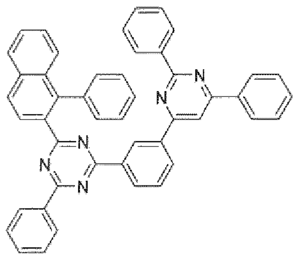

ET-5

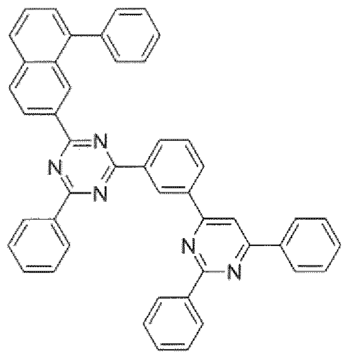

ET-6

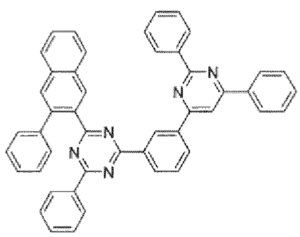

ET-7

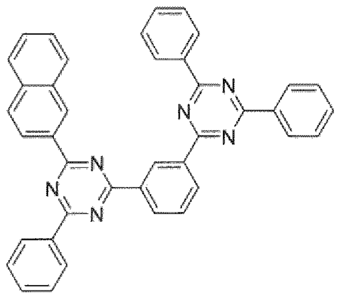

ET-8

<u>OLED performance</u>

*For layer Stack A:*

| ETL | CIEy | Rel. Voltage (%) V @ [10 mA/cm$^2$] | Rel. CEff/CIEy (%) cd/A @ [10 mA/cm$^2$] |
|---|---|---|---|
| C-1 (Comp. ex.) | 0.052 | 100% | 100% |
| ET-1 | 0.053 | 95% | 112% |
| ET-3 | 0.053 | 95% | 112% |
| ET-5 | 0.053 | 95% | 112% |
| ET-6 | 0.051 | 95% | 115% |
| ET-7 | 0.053 | 95% | 113% |
| ET-8 | 0.050 | 94% | 116% |

*For layer stack B:*

| ETL | CIEy | Rel. Voltage (%) (V) V @ [10 mA/cm$^2$] | Rel. CEff/CIEy (%) cd/A @ [10 mA/cm$^2$] |
|---|---|---|---|
| C-1:LiQ (Comp. Ex.) | 0.052 | 100% | 100% |
| ET-1:LiQ | 0.052 | 98% | 107% |
| ET-3:LiQ | 0.053 | 9896 | 107% |
| ET-5:LiQ | 0.052 | 9996 | 108% |
| ET-6:LiQ | 0.052 | 98% | 107% |
| ET-7:LiQ | 0.053 | 99% | 108% |
| ET-8:LiQ | 0.052 | 97% | 108% |

The features disclosed in the foregoing description and in the dependent claims may, both separately and in any combination thereof, be material for realizing the aspects of the disclosure made in the independent claims, in diverse forms thereof.

## Claims

**1.** A compound of formula (I)

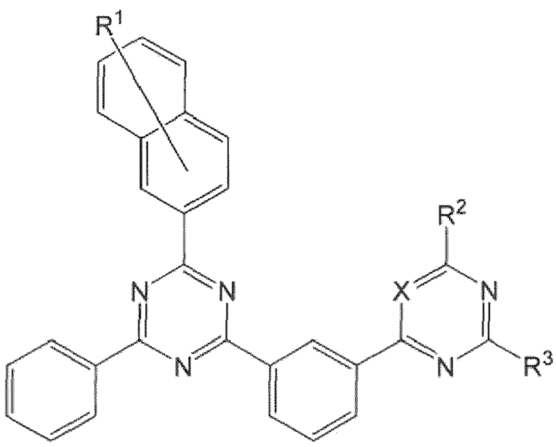

(I)

wherein

- X is CH or N;

- $R^1$ is selected from the group consisting of H, phenyl, biphenyl, pyridyl, naphthyl, dibenzofuranyl, and dibenzothiophenyl;
- $R^2$ and $R^3$ are independently $C_6$ to $C_{12}$;
- at least one of $R^2$ and $R^3$ is phenyl; and
- the compound of formula (I) may be unsubstituted or substituted with one or more substituents independently selected from the group consisting of D, $C_1$ to $C_6$ alkyl; $C_1$ to $C_6$ alkoxy; and $SiR'_3$, wherein R' is independently selected from $C_1$ to $C_6$ alkyl.

**2.** The compound according to claim 1, wherein X is N.

**3.** The compound according to claim 1 or 2, wherein $R^1$ is selected from the group consisting of phenyl, biphenyl, pyridyl, alkyl, naphthyl, dibenzofuranyl, and dibenzothiophenyl.

**4.** The compound according to claim 1 or 2, wherein $R^1$ is selected from the group consisting of H, phenyl, biphenyl, pyridyl, and dibenzofuranyl.

**5.** The compound according to claim 1, 2, or 4, wherein $R^1$ is selected from the group consisting of H and phenyl.

**6.** The compound according to any of the preceding claims, wherein

- $R^2$ is phenyl and $R^3$ is phenyl; or
- $R^2$ is phenyl and $R^3$ is biphenyl.

**7.** The compound according to any of the preceding claims, wherein $R^2$ is phenyl and $R^3$ is phenyl.

**8.** The compound according to any of the preceding claims, wherein the compound of formula (I) is selected from ET-1 to ET-22

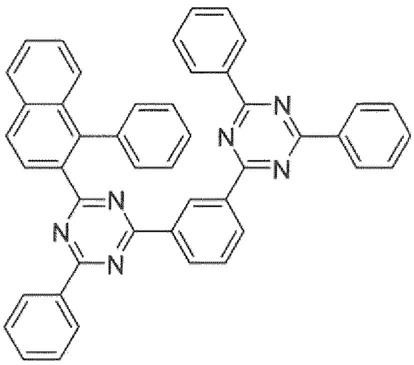
ET-1

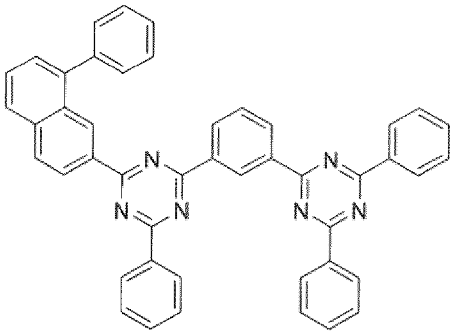
ET-2

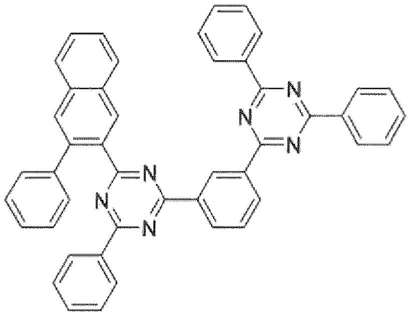
ET-3

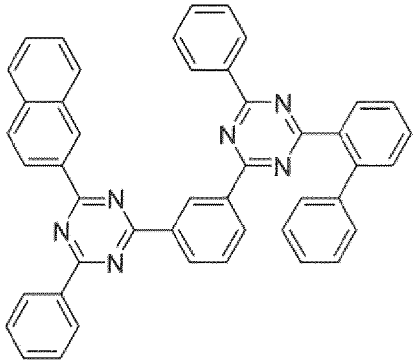
ET-4

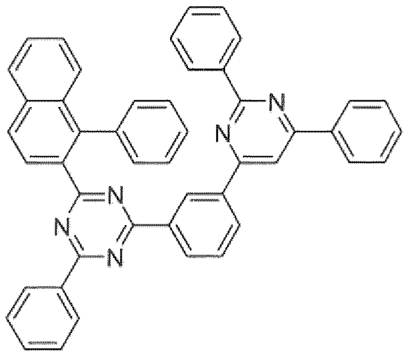

ET-5

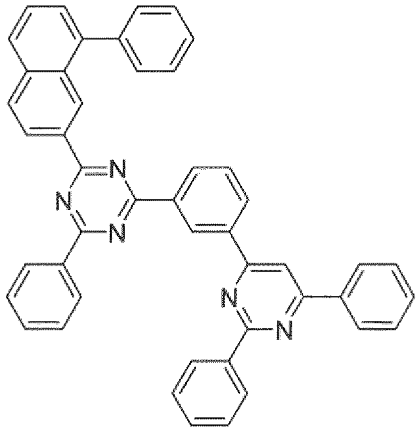

ET-6

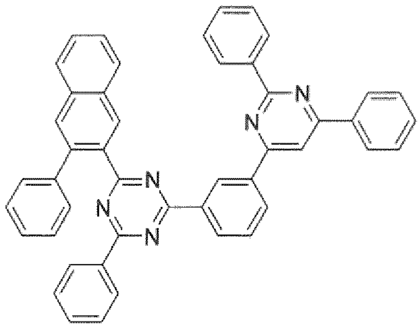

ET-7

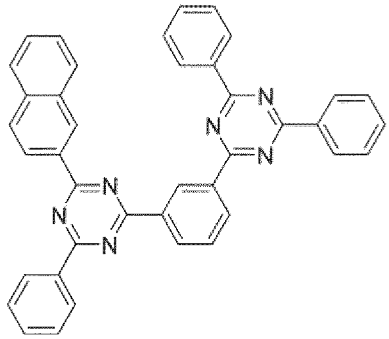

ET-8

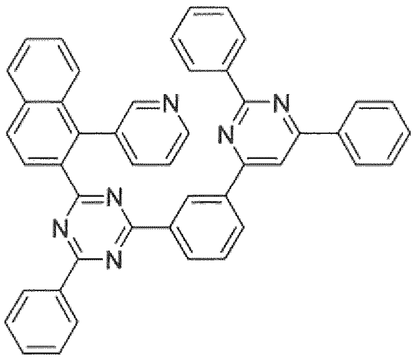

ET-9

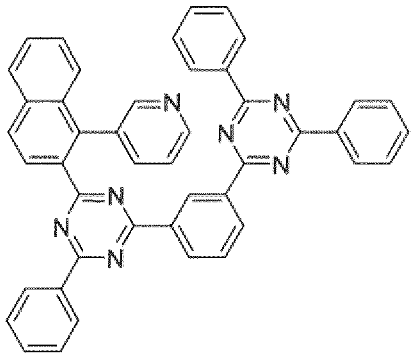

ET-10

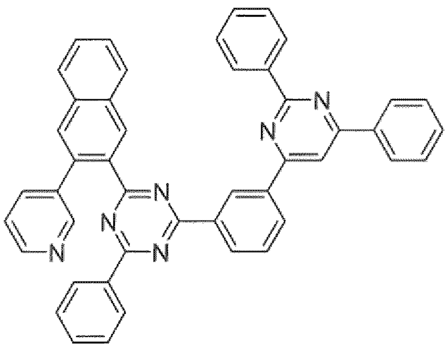

ET-11

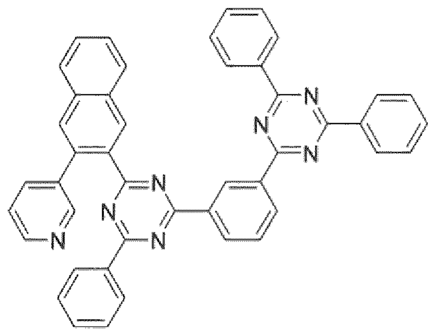

ET-12

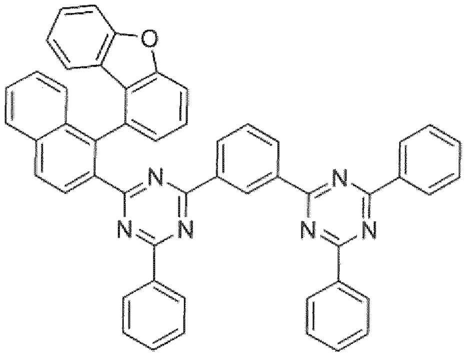
ET-13

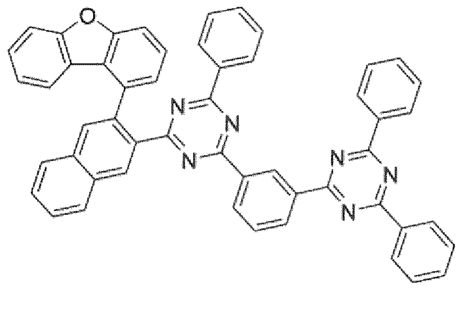
ET-14

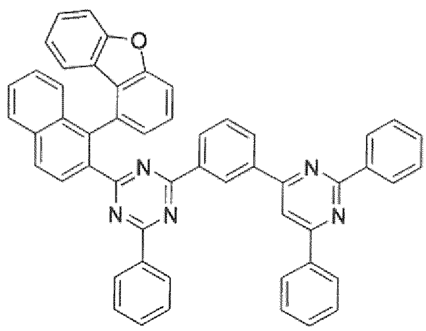
ET-15

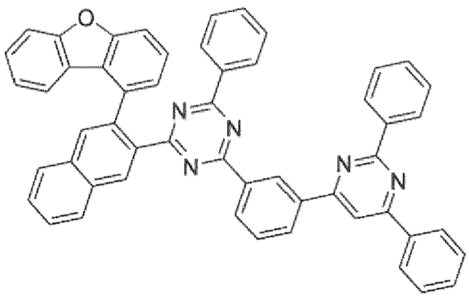
ET-16

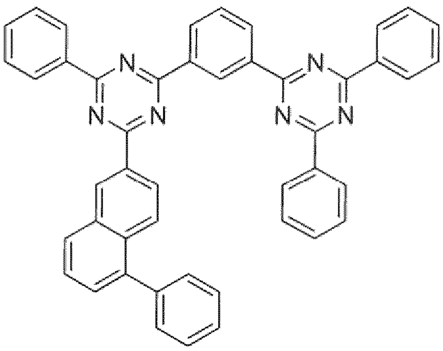
ET-17

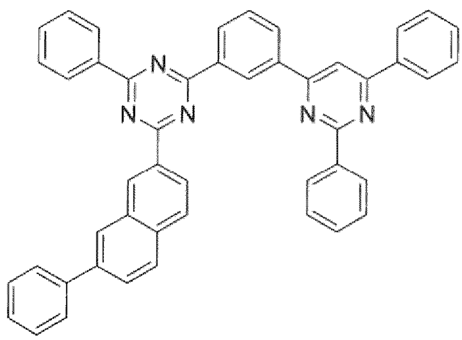
ET-18

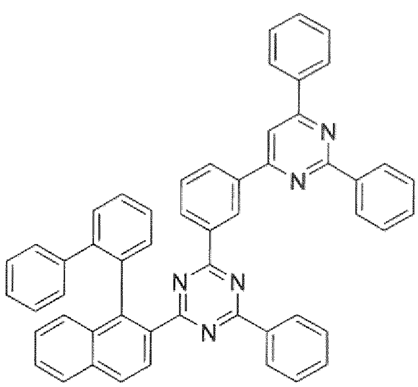
ET-19

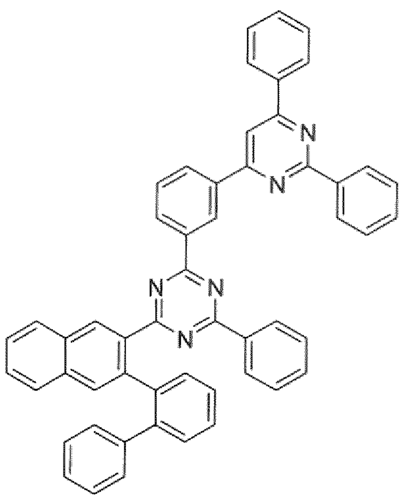
ET-20

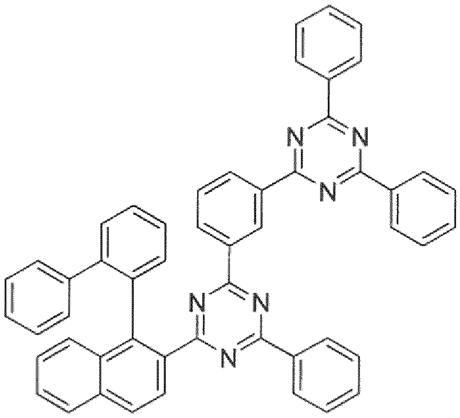

ET-21

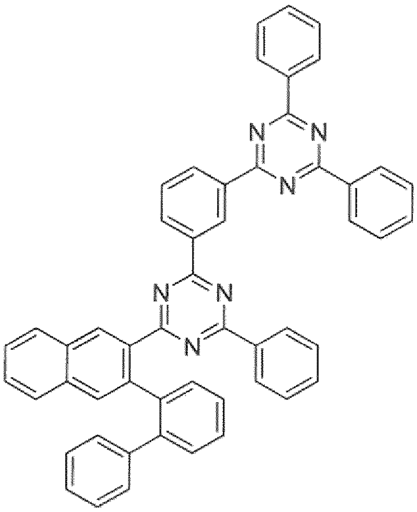

ET-22.

9. An organic light emitting diode comprising an anode, a cathode, and an organic semiconducting layer, wherein

- the organic semiconducting layer is arranged between the anode and the cathode; and
- the organic semiconducting layer comprises the compound of formula (I) according to any of the preceding claims.

10. The organic light emitting diode according to claim 9, wherein the organic semiconducting layer consists of the compound of formula (I).

11. The organic light emitting diode according to claim 9, wherein the organic semiconducting layer further comprises an electrical n-dopant.

12. The organic light emitting diode according to any of the claims 9 to 11, wherein the organic semiconducting layer is an electron transport layer.

13. The organic light emitting diode according to any of the claims 9 to 12, wherein the organic light emitting diode further comprises a light emitting layer, and the organic semiconducting layer is arranged between the light emitting layer and the cathode.

14. The organic light emitting diode according to claim 13, wherein the organic light emitting diode further comprises an auxiliary electron transport layer and the auxiliary electron transport layer is arranged between the light emitting layer and the organic semiconducting layer.

15. Display device comprising the organic light emitting diode according to any of the claims 9 to 14.

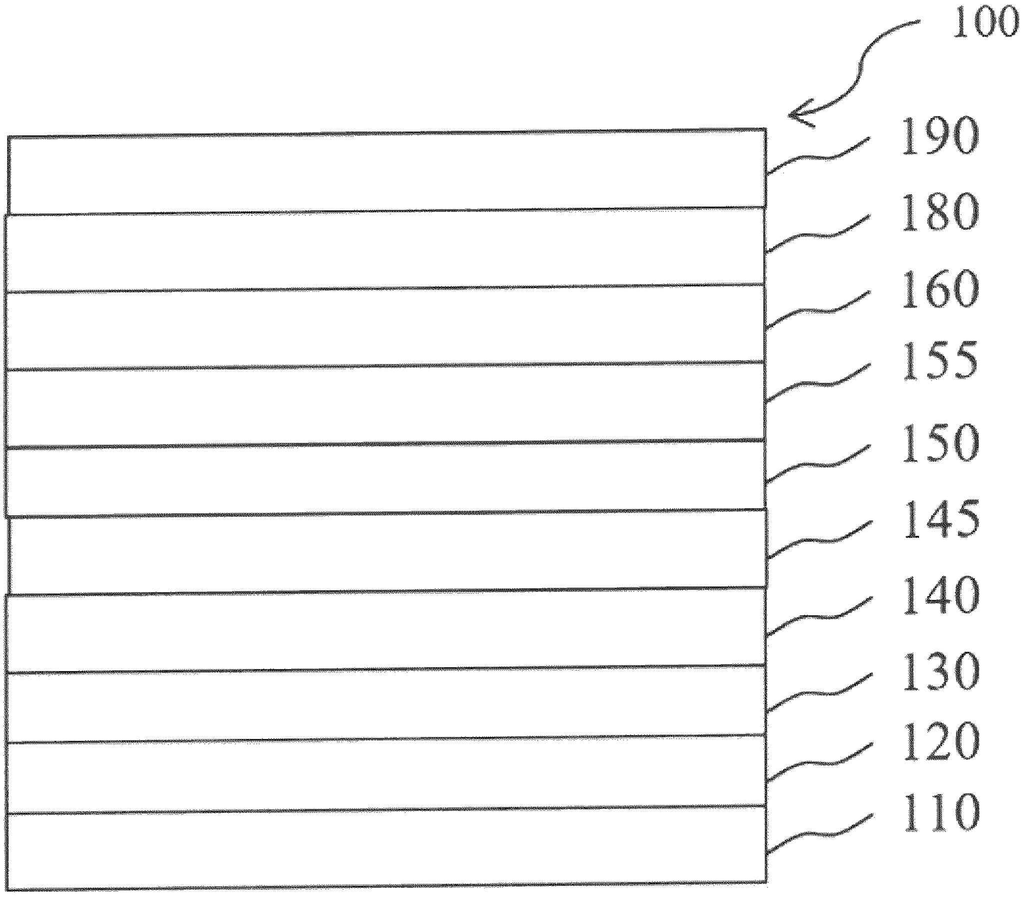


Fig. 1

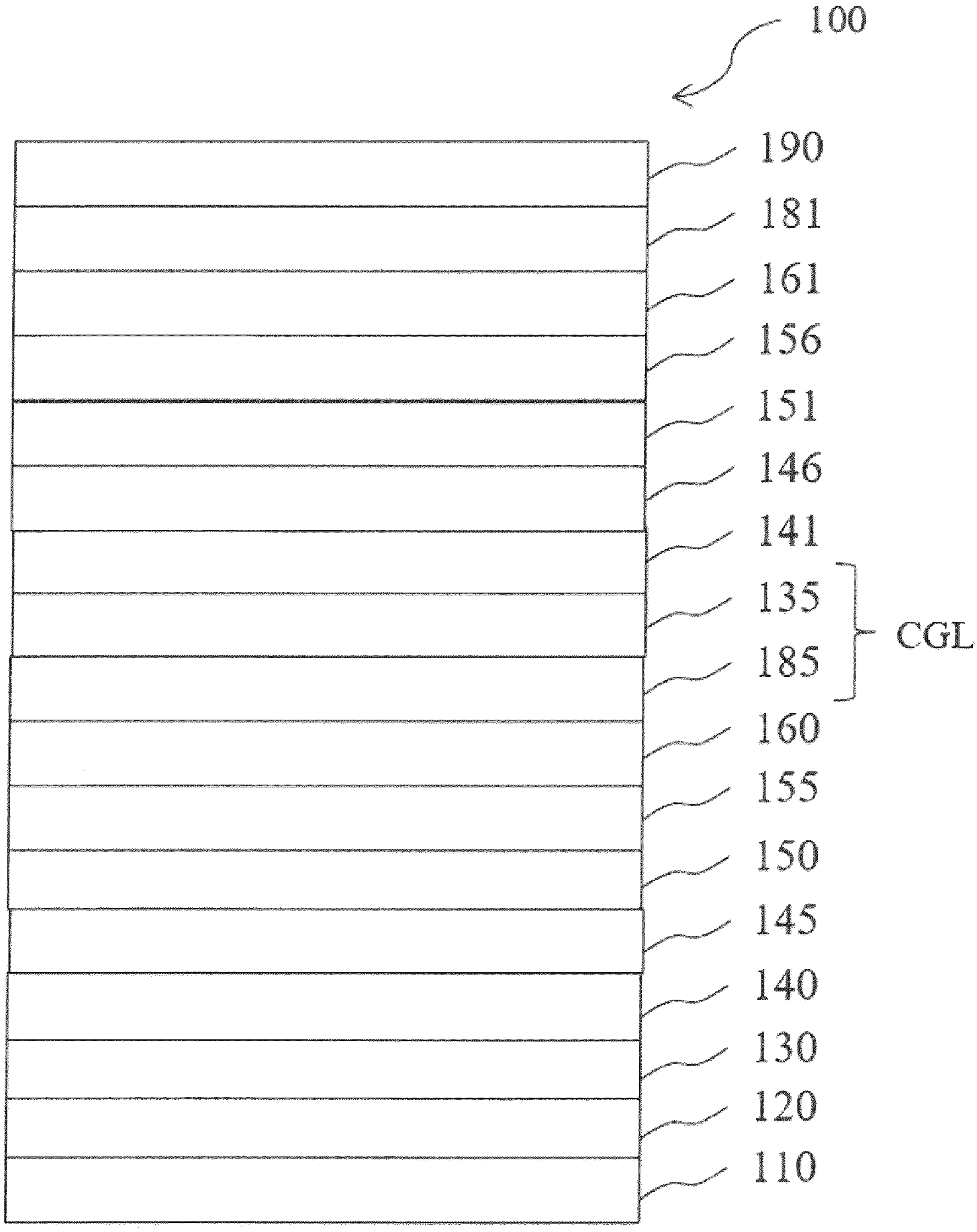
100
190
181
161
156
151
146
141
135
185
CGL
160
155
150
145
140
130
120
110

Fig. 2

Europäisches Patentamt
European Patent Office
Office européen des brevets

# EUROPEAN SEARCH REPORT

Application Number
EP 24 21 7166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 112 409 276 A (BEIJING ETERNAL MAT TECH CO LTD) 26 February 2021 (2021-02-26)<br>* Compounds C3, C8, C33, C38, C63 => extra CN, F , CF3 substituent on central Ph;<br>claims 1-11 *<br>----- | 1-15 | INV.<br>C07D251/24<br>H10K50/10 |
| X | US 2024/292743 A1 (SHIN HYUN [KR] ET AL) 29 August 2024 (2024-08-29)<br>* Compounds 022, 023, 098, 122, 123<br>Extra C7 (vs C8) substituent on R2/R3;<br>claims 1-20 *<br>----- | 1-15 | |
| X | WO 2019/098695 A1 (DOOSAN CORP [KR]) 23 May 2019 (2019-05-23)<br>* Page 52 compound 91 => extra pyridine on phenyl ring *<br>----- | 1-15 | |

TECHNICAL FIELDS SEARCHED (IPC)
C07D
H10K

The present search report has been drawn up for all claims

1

| Place of search | Date of completion of the search | Examiner |
|---|---|---|
| Munich | 23 May 2025 | Sotoca Usina, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 21 7166

This annex lists the patent family members relating to the patent documents cited in the above-mentioned European search report. The members are as contained in the European Patent Office EDP file on
The European Patent Office is in no way liable for these particulars which are merely given for the purpose of information.

23-05-2025

| Patent document cited in search report | Publication date | Patent family member(s) | Publication date |
|---|---|---|---|
| CN 112409276 A | 26-02-2021 | NONE | |
| US 2024292743 A1 | 29-08-2024 | CN 118459415 A | 09-08-2024 |
| | | KR 20240123885 A | 16-08-2024 |
| | | US 2024292743 A1 | 29-08-2024 |
| WO 2019098695 A1 | 23-05-2019 | CN 111406052 A | 10-07-2020 |
| | | CN 117304173 A | 29-12-2023 |
| | | KR 20190055538 A | 23-05-2019 |
| | | WO 2019098695 A1 | 23-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005086251 A
- EP 1837926 B1
- WO 2007107306 A
- WO 2007107356 A
- EP 2722908 A1
- EP 1970371 A1
- WO 2013079217 A1
- US 2016322581 A
- US 2008265216 A
- WO 2016105141 A
- JP 2004281390 B
- WO 2013079217 A


### Non-patent literature cited in the description


- **YASUHIKO SHIROTA** ; **HIROSHI KAGEYAMA**. *Chem. Rev.*, 2007, vol. 107, 953-1010
- *CHEMICAL ABSTRACTS*, 1242056-42-3
- *CHEMICAL ABSTRACTS*, 1613079-70-1
- *CHEMICAL ABSTRACTS*, 1224447-88-4
- *CHEMICAL ABSTRACTS*, 1955543-57-3
- *CHEMICAL ABSTRACTS*, 721969-94-4
- *CHEMICAL ABSTRACTS*, 850918-68-2